# EUROPEAN PATENT APPLICATION

(11) **EP 4 851 280 A1**
(43) Date of publication of application: **09.12.2026**
(21) Application number: 25755106.9
(22) Date of filing: 14.02.2025
(51) Int. Cl.: C12N 5/078, C12M 3/00, C12N 5/0775, C12N 5/10

(54) **BONE-FORMING COMPOSITION**

(30) Priority: 14.02.2024 JP 2024019962
(71) Applicant: NAGASAKI UNIVERSITY, Nagasaki 852-8521 (JP); Megakaryon Corporation, Kyoto-shi, Kyoto 600-8813 (JP)
(72) Inventor: SUMITA, Yoshinori, Nagasaki-shi, Nagasaki 852-8521 (JP); OGAERI, Takunori, Nagasaki-shi, Nagasaki 852-8521 (JP); NAGANO, Toshiki, Nagasaki-shi, Nagasaki 852-8521 (JP); ASAMI, Asano, Kyoto-shi, Kyoto 600-8813 (JP); TOMIZUKA, Junko, Kyoto-shi, Kyoto 600-8813 (JP); HIROSE, Hidenori, Kyoto-shi, Kyoto 600-8813 (JP)
(74) Representative: Dr. Schön, Neymeyr & Partner Patentanwälte mbB
(86) International application number: PCT/JP2025/005038
(87) International publication number: WO 2025/173790

(57) **Abstract**

An object is to provide a bone-forming composition comprising enlarged megakaryocytes and/or extracellular secretion products derived from enlarged megakaryocytes. The bone-forming composition of the present disclosure comprises enlarged megakaryocytes and/or extracellular secretion products derived from enlarged megakaryocytes.

## Description

### TECHNICAL FIELD

The present disclosure relates to a bone-forming composition.

### BACKGROUND ART

Extracellular secretion products such as secretomes and extracellular vesicles are known to be involved in intercellular and inter-organ information transmission, functional regeneration of tissues, and the like (Patent Document 1, Non-Patent Documents 1-2). Application of the extracellular secretion products to regenerative medicine and the like has been explored.

Furthermore, in regenerative medicine in the dental field, platelet-rich plasma has been used in bone augmentation treatments and the like. However, since the therapeutic application of the platelet-rich plasma requires preparation at the time of use, a process from blood collection to production is required during surgery, and its operation is complicated, which is problematic. Moreover, since the platelet-rich plasma is prepared by separation and centrifugal concentration from isolated human blood, the platelet count is not uniform, and its efficacy is often uncertain, which constitutes an obstacle in use (Non-Patent Document 3).

### CITATION LIST

### Patent Document

Patent Document 1: WO 2010/079086

### Non-Patent Literature

Non-Patent Document 1: Beer, Lucian et al. "Peripheral blood mononuclear cell secretome for tissue repair." Apoptosis : an international journal on programmed cell death vol. 21,12 (2016): 1336-1353. doi:10.1007/s10495-016-1292-8
Non-Patent Document 2: Gonzalez-Gonzalez, Alberto et al. "Mesenchymal stem cells secretome: The cornerstone of cell-free regenerative medicine." World journal of stem cells vol. 12,12 (2020): 1529-1552. doi:10.4252/wjsc.v12.i12.1529
Non-Patent Document 3: Fernandes, G. & Yang, S. "Application of platelet-rich plasma with stem cells in bone and periodontal tissue engineering." Bone Res., 4 (2016): 16036. doi:10.1038/boneres.2016.36. eCollection 2016

### SUMMARY OF INVENTION

### Problem to be Solved by the Invention

Therefore, an object of the present disclosure is to provide a novel composition having megakaryocyte-derived bone-forming activity.

### Solution to Problem

In order to achieve the object, the bone-forming composition of the present disclosure (hereinafter also referred to as the "composition") comprises an enlarged megakaryocyte and/or an extracellular secretion product derived from an enlarged megakaryocyte.

The bone-forming kit of the present disclosure (hereinafter also referred to as the "kit") comprises a bone-forming composition and a scaffold material for bone formation, wherein the bone-forming composition is the bone-forming composition of the present disclosure.

### Advantageous Effects of Invention

According to the present disclosure, a novel composition having megakaryocyte-derived bone-forming activity can be provided. In addition, the composition can be provided as an off-the-shelf product that does not require blood collection or preparation.

### BRIEF DESCRIPTION OF DRAWINGS

[FIG. 1] FIG. 1 is a photograph showing bone formation four weeks after transplantation in Example 1.
[FIG. 2] FIG. 2 is a graph showing bone volume four weeks after transplantation in Example 1.
[FIG. 3] FIG. 3 is a photograph showing bone formation four weeks after transplantation in Example 1.
[FIG. 4] FIG. 4 is a graph showing bone volume four weeks after transplantation in Example 1.
[FIG. 5] FIG. 5 is a graph showing cell number in Example 1.

### DESCRIPTION OF EMBODIMENTS

### <Bone-Forming Composition>

As described above, the bone-forming composition of the present disclosure comprises enlarged megakaryocytes and/or an extracellular secretion product derived from enlarged megakaryocytes.

In the present disclosure, "bone formation" may be used to mean any of the following: an increase in already-existing bone, that is, "bone augmentation"; the formation of new bone, that is, "new bone formation"; or the recovery of lost (defective) bone or a portion thereof, that is, "bone regeneration." Therefore, the bone-forming composition of the present disclosure can also be referred to as a bone augmentation composition, a new bone formation composition, and/or a bone regeneration composition.

In the present disclosure, "secretome" means a group of molecules secreted by cells. The group of molecules includes, for example, proteins, extracellular vesicles, and the like. The proteins include, for example, growth factors, cytokines, chemokines, adhesion molecules, proteases, and the like.

In the present disclosure, "extracellular vesicle (EV)" means a particle surrounded by a lipid bilayer membrane secreted by cells. The extracellular vesicle contains, for example, nucleic acid molecules, growth factors, proteins, and the like. The nucleic acid molecules include, for example, DNA, mRNA, miRNA, and the like. The extracellular vesicles include, for example, exosomes, microvesicles, and the like.

In the present disclosure, "extracellular secretion product" means, for example, substances secreted by cells such as the secretome and the extracellular vesicles.

In the present disclosure, a "megakaryocyte" (megakaryocyte cell) is the largest cell present in the bone marrow or the like *in vivo*, and means a cell capable of releasing platelets, a cell that releases platelets, and a cell having an equivalent function. The cell having an equivalent function means a cell having platelet-producing ability. In the present disclosure, the megakaryocyte may be a pre-multinucleated (polyploid) megakaryocyte, that is, an immature megakaryocyte or a proliferating megakaryocyte, may be a megakaryocyte after multinucleation (a multinucleated megakaryocyte), or may be a megakaryocyte after enlargement (an enlarged megakaryocyte). The multinucleated megakaryocyte can also be referred to as, for example, a megakaryocyte that has undergone multinucleation but in which enlargement has not occurred. As a specific example, the megakaryocyte may be any of, for example, a promegakaryoblast, a megakaryoblast, a promegakaryocyte, and a mature megakaryocyte. The ploidy (N) of the megakaryocyte after multinucleation may be greater than 2 sets, and specific examples thereof are 4 to 128 sets (4 to 128N), 4 to 64 sets (4N to 64N), 4 to 32 sets (4N to 32N), 8 to 128 sets (8 to 128N), 8 to 64 sets (8 to 64N), 8 to 32 sets (8N to 32N), 16 to 128 sets (16 to 128N), 16 to 64 sets (16 to 64N), or 16 to 32 sets (16 to 32N).

The origin of the megakaryocyte is not particularly limited, and examples thereof include humans and non-human animals. Examples of the non-human animal include primates such as monkeys, gorillas, chimpanzees, and marmosets, as well as mice, rats, dogs, cats, rabbits, sheep, horses, guinea pigs, and the like.

In the present disclosure, the megakaryocyte can be identified by cell surface markers. When the megakaryocyte is derived from a human, examples of the cell surface markers include CD41a, CD42a, and CD42b. That is, the megakaryocyte is a cell positive for CD41a, CD42a, and CD42b. When the megakaryocyte is derived from a human, the cell surface marker may be, for example, at least one selected from the group consisting of CD9, CD61, CD62p, CD42c, CD42d, CD49f, CD51, CD110, CD123, CD131, and CD203c.

The pre-multinucleated megakaryocyte and the multinucleated megakaryocyte can be identified, for example, by the ploidy (N). As a specific example, in a target megakaryocyte, the target megakaryocyte having a ploidy (N) of less than 8N can be evaluated as a pre-multinucleated megakaryocyte. On the other hand, in a target megakaryocyte, the target megakaryocyte having a ploidy (N) of 8N or more, preferably of 16N or more, can be evaluated as a multinucleated megakaryocyte.

When there are a plurality of the target megakaryocytes, that is, when there is a population of the target megakaryocytes (a cell population), in the population of the target megakaryocytes, when the proportion of cells having a ploidy (N) of 8N or more is less than 10%, the population of the target megakaryocytes can be evaluated as a population of pre-multinucleated megakaryocytes. Furthermore, in the population of the target megakaryocytes, when the proportion of cells having a ploidy (N) of 8N or more is 20% or more, 21% or more, 22% or more, 23% or more, 24% or more, 25% or more, 26% or more, 27% or more, 28% or more, 29% or more, 30% or more, 35% or more, 40% or more, 45% or more, or 50% or more, the population of the target megakaryocytes can be evaluated as a population containing multinucleated megakaryocytes. The population of the target megakaryocytes is, for example, from 1×10⁵ to 1×10⁶ cells.

In the present disclosure, an "enlarged megakaryocyte" means a megakaryocyte in which the cytoplasm of the multinucleated megakaryocyte has enlarged. The enlarged megakaryocyte contains, for example, alpha-granules. The ploidy (N) of the enlarged megakaryocyte is the same as the ploidy of the multinucleated megakaryocyte before enlargement. Therefore, the ploidy (N) of the enlarged megakaryocyte may be greater than 2 sets, and specific examples thereof are 4 to 128 sets (4N to 128N), 4 to 64 sets (4N to 64N), 4 to 32 sets (4N to 32N), 8 to 128 sets (8N to 128N), 8 to 64 sets (8 to 64N), 8 to 32 sets (8N to 32N), 16 to 128 sets (16N to 128N), 16 to 64 sets (16 to 64N), or 16 to 32 sets (16 to 32N).

In the present disclosure, the enlarged megakaryocyte can be identified, for example, by markers. When the enlarged megakaryocyte is derived from a human-derived immortalized megakaryocyte, examples of the markers include Ki-67 and/or MYC. In the enlarged megakaryocyte, for example, compared with the multinucleated megakaryocyte, the expression level of the marker is reduced to 50% or less, 40% or less, 30% or less, 20% or less, 10% or less, 5% or less, 4% or less, 3% or less, 2% or less, 1% or less, 0.1% or less, or 0.01% or less. Accordingly, when the target megakaryocytes include a plurality of megakaryocytes, that is, when there is a population of the target megakaryocytes (a cell population), in the population of the target megakaryocytes, when the expression of the marker is reduced to 50% or less, 40% or less, 30% or less, 20% or less, 10% or less, 5% or less, 4% or less, 3% or less, 2% or less, 1% or less, 0.1% or less, or 0.01% or less compared with the multinucleated megakaryocyte, the population of megakaryocytes can be evaluated as a population containing enlarged megakaryocytes. The population of the target megakaryocytes is, for example, from 1×10⁵ to 1×10⁶ cells. One type of the marker may be used, or a plurality of types may be used. When a plurality of types of markers are used, the combination of the markers is, for example, Ki-67 and/or MYC.

The enlarged megakaryocyte can also be distinguished, for example, by cell size. Among the megakaryocytes, the multinucleated megakaryocyte is, for example, relatively larger in cell size than an immature megakaryocyte or a proliferating megakaryocyte. Furthermore, the enlarged megakaryocyte is, for example, relatively larger in cell size than the multinucleated megakaryocyte. As an example, the cell size of the immature megakaryocyte or the proliferating megakaryocyte is, for example, equal to or less than 10 µm. The cell size of the multinucleated megakaryocyte is, for example, greater than 10 µm and 60 µm or less. The cell size of the enlarged megakaryocyte is, for example, greater than 50 µm and 100 µm or less. As for the cell size, it is preferable, for example, to measure the major axis. The measurement is preferably performed by observation under a light microscope with an objective lens magnification of 10 to 20 times.

In the present disclosure, the enlarged megakaryocyte may be composed, for example, only of enlarged megakaryocytes, or may include other cells. In the latter case, the enlarged megakaryocyte can also be referred to as, for example, a cell population containing enlarged megakaryocytes. The enlarged megakaryocyte may be, for example, an enlarged megakaryocyte of 8N or more, an enlarged megakaryocyte of 16N or more, or an enlarged megakaryocyte of 32N or more. The enlarged megakaryocytes include, for example, the enlarged megakaryocyte of 8N or more, the enlarged megakaryocyte of 16N or more, and/or the enlarged megakaryocyte of 32N or more.

When the enlarged megakaryocytes include enlarged megakaryocytes having a ploidy of 8N or more, in the cell population, the lower limit value of the proportion (cell number) of the enlarged megakaryocytes of8N or more is, for example, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 35% or more, 40% or more, 45% or more, 50% or more, 55% or more, or 60% or more. In the cell population, the upper limit value of the proportion (cell number) of the enlarged megakaryocytes oof 8N or more is, for example, equal to or less than 90% or less, 85% or less, 80% or less, 75% or less, 70% or less, 65% or less, 60% or less, 59% or less, 58% or less, 57% or less, 56% or less, 55% or less, 54% or less, 53% or less, 52% or less, 51% or less, 50% or less, 49% or less, 48% or less, 47% or less, or 46% or less. The proportion (cell number) of the enlarged megakaryocytes of 8N or more is, for example, 15% to 90%, 20% to 90%, 25% to 90%, 30% to 90%, 35% to 90%, 40% to 90%, 45% to 90%, 50% to 90%, 55% to 90%, 60% to 90%, 15% to 85%, 15% to 80%, 15% to 75%, 15% to 70%, 15% to 65%, 16% to 60%, 17% to 59%, 18% to 58%, 19% to 57%, 20% to 56%, 21% to 55%, 22% to 54%, 23% to 54%, 24% to 53%, 25% to 51%, 26% to 50%, 27% to 49%, 28% to 48%, 29% to 47%, or 30% to 46%. The proportion (cell number) can be measured, for example, by sufficiently suspending the target cell population, then taking a portion of the resulting cell suspension, and measuring it by a method for measuring the ploidy (the same applies hereinafter).

When the enlarged megakaryocytes include enlarged megakaryocytes having a ploidy of 16N or more, in the cell population, the lower limit value of the proportion (cell number) of the enlarged megakaryocytes of 16N or more is, for example, 15% or more, 16% or more, 17% or more, 18% or more, 19% or more, 20% or more, 21% or more, 22% or more, 23% or more, 24% or more, or 25% or more. In the cell population, the upper limit value of the proportion (cell number) of the enlarged megakaryocytes of 16N or more is, for example, 90% or less, 85% or less, 80% or less, 75% or less, 70% or less, 65% or less, 60% or less, 55% or less, 54% or less, 53% or less, 52% or less, 51% or less, 50% or less, 49% or less, 48% or less, 47% or less, 46% or less, 45% or less, 44% or less, or 43% or less. The proportion (cell number) of the enlarged megakaryocytes of 16N or more is, for example, 15% to 90%, 15% to 85%, 15% to 80%, 15% to 75%, 15% to 70%, 15% to 65%, 15% to 60%, 16% to 55%, 17% to 54%, 18% to 53%, 19% to 52%, 20% to 90%, 20% to 70%, 20% to 51%, 21% to 50%, 22% to 49%, 23% to 48%, 24% to 47%, 25% to 46%, 25% to 45%, 25% to 44%, or 25% to 43%.

When the enlarged megakaryocytes include enlarged megakaryocytes having a ploidy of 32N or more, in the cell population, the lower limit value of the proportion (cell number) of the enlarged megakaryocytes of 32N or more is, for example, 5% or more, 6% or more, 7% or more, 8% or more, 9% or more, 10% or more, 11% or more, 12% or more, 13% or more, 14% or more, or 15% or more. In the cell population, the upper limit value of the proportion (cell number) of the enlarged megakaryocytes of 32N or more is, for example, 50% or less, 49% or less, 48% or less, 47% or less, 46% or less, 45% or less, 44% or less, 43% or less, 42% or less, 41% or less, 40% or less, 39% or less, 38% or less, 37% or less, 36% or less, 35% or less, 34% or less, 33% or less, 32% or less, 31% or less, or 30% or less. The proportion (cell number) of the enlarged megakaryocytes equal to or greater than 32N is, for example, 5% to 50%, 5% to 49%, 5% to 48%, 5% to 47%, 5% to 46%, 5% to 45%, 5% to 44%, 5% to 43%, 5% to 42%, 5% to 41%, 5% to 40%, 6% to 39%, 7% to 38%, 8% to 37%, 9% to 36%, 10% to 35%, 11% to 34%, 12% to 33%, 13% to 32%, 14% to 31%, 15% to 31%, or 15% to 30%.

The enlarged megakaryocyte is, for example, a cell derived from a pluripotent cell, and is preferably a cell induced from a pluripotent cell *in vitro*. The enlarged megakaryocyte is preferably derived, for example, from an immortalized megakaryocyte. The immortalized megakaryocyte can be prepared, for example, by introducing an oncogene, a polycomb gene, and/or an anti-apoptotic gene, as described below. Therefore, the megakaryocyte before enlargement (the multinucleated megakaryocyte) and the enlarged megakaryocyte contain, for example, an exogenous oncogene, polycomb gene, and/or anti-apoptotic gene. The oncogene is preferably the c-MYC gene. The polycomb gene is preferably the BMI1 gene. The anti-apoptotic gene is preferably the BCL-XL gene. The megakaryocyte before enlargement and the enlarged megakaryocyte, for example, express an exogenous oncogene, polycomb gene, and/or anti-apoptotic gene.

The bone-forming composition of the present disclosure may contain, for example, factors such as growth factors. Examples of the growth factors include basic fibroblast growth factor (bFGF), platelet-derived growth factor-BB (PDGF-BB), and the like. Examples of the growth factors include transforming growth factor-beta (TGF-β), vascular endothelial growth factor (VEGF), and the like. The content of the factor in the composition can be defined, for example, as a concentration or as the content in a composition prepared from a predetermined amount of cells.

The content of the factor in the composition can be evaluated, for example, based on a composition prepared using 1×10⁶ enlarged megakaryocytes described below. When converted to a composition prepared from 1×10⁶ enlarged megakaryocytes, the range of the content of the bFGF is, for example, 1000 to 100000 pg, 2000 to 80000 pg, or 3000 to 71000 pg. The range of the content of the PDGF-BB is, for example, 1000 to 50000 pg, 2000 to 40000 pg, or 3000 to 30000 pg. The range of the content of the TGF-β is, for example, 20 to 3000 pg, 30 to 2000 pg, or 35 to 1500 ng. The range of the content of the VEGF is, for example, 50 to 7000 pg, 65 to 5000 pg, or 80 to 4500 pg.

The enlarged megakaryocyte may be produced, for example, by a production method including a multinucleation step of multinucleating a pre-multinucleated megakaryocyte or a progenitor cell thereof to induce a multinucleated megakaryocyte, and an enlargement step of maturing the multinucleated megakaryocyte to induce an enlarged megakaryocyte. Furthermore, in the multinucleation step, the activity of at least one selected from the group consisting of an aryl hydrocarbon receptor (AhR), a Rho-associated coiled-coil forming kinase (ROCK), a dual-specificity tyrosine phosphorylation-regulated kinase (DYRK), and myosin 2 may be inhibited, and, in the presence of harmine, the pre-multinucleated megakaryocyte may be multinucleated to induce a multinucleated megakaryocyte. According to the present disclosure, by inducing enlarged megakaryocytes using the multinucleated megakaryocytes induced in the multinucleation step, the amount of the secretome and the extracellular vesicles secreted from the enlarged megakaryocytes can be improved. Thereby, the present disclosure can, for example, increase the content of the secretome, the extracellular vesicles, and/or the factors in the cultured medium. A factor that promotes the proliferation of the pre-multinucleated megakaryocyte, described below, may be present in the cultured medium, or may be present within the pre-multinucleated megakaryocyte. The activity-inhibiting substance for the AhR (an AhR inhibitor), the activity-inhibiting substance for the ROCK (a ROCK inhibitor), the activity-inhibiting substance for the DYRK (a DYRK inhibitor), the activity-inhibiting substance for the myosin 2 (a myosin 2 inhibitor), and/or harmine are contained, for example, in the medium of the pre-multinucleated megakaryocyte, described below. When the factor that promotes the proliferation of the pre-multinucleated megakaryocyte, the activity-inhibiting substance for the AhR, the activity-inhibiting substance for the ROCK, the activity-inhibiting substance for the DYRK, and/or the activity-inhibiting substance for the myosin 2 is a protein or a nucleic acid molecule, in the multinucleation step, it may be introduced into the pre-multinucleated megakaryocyte by lipofection, electroporation, or the like.

In the multinucleation step, the activity of any one of AhR, ROCK, DYRK, and myosin 2 may be inhibited, or the activity of two or more thereof may be inhibited. In the latter case, the combination of the targets whose activity is to be inhibited may be any combination, and, because of the high multinucleation-promoting ability, it is preferably a combination of AhR and myosin 2, a combination of AhR, ROCK, and myosin 2, a combination of AhR, DYRK, and myosin 2, or a combination of AhR, ROCK, DYRK, and myosin 2. Therefore, the multinucleation step is preferably carried out by inhibiting the activity of AhR and myosin 2 in the presence of harmine, by inhibiting the activity of AhR, ROCK, and myosin 2 in the presence of harmine, by inhibiting the activity of AhR, DYRK, and myosin 2 in the presence of harmine, or by inhibiting the activity of AhR, ROCK, DYRK, and myosin 2 in the presence of harmine.

The AhR means an aryl hydrocarbon receptor. The AhR is a transcription factor belonging to the Per/ARNT/SIM (PAS) family. The AhR is, for example, inactive in a state in which no ligand is bound, and translocates into the nucleus when an aromatic hydrocarbon compound binds thereto as a ligand. Then, after the nuclear translocation, the AhR forms, for example, a heterodimer with ARNT (AhR Nuclear Translocator), binds to a Xenobiotic Responsive Element (XRE, also referred to as DRE), and activates transcription.

In the multinucleation step, the inhibition of the activity of AhR means that the activity of AhR is reduced compared with the case in the absence of the activity-inhibiting substance for the AhR. The activity of the AhR can be measured, for example, using the nuclear translocation of AhR as an index. The activity of the AhR can be measured, for example, using a commercially available kit for measuring the activity of AhR protein, and specific examples thereof include a nuclear receptor assay kit (manufactured by PURACYP). In the present disclosure, the inhibition of the activity can also be referred to as, for example, inhibition of the activity, reduction of the activity, suppression of the activation, prevention of the activation, or the like.

The AhR inhibitor is not particularly limited, and examples thereof include an AhR antagonist, an expression-suppressing nucleic acid molecule capable of suppressing the expression of AhR, and the like. The AhR antagonist is not particularly limited, and examples thereof include 4-(2-(2-(Benzo[b]thiophen-3-yl)-9-isopropyl-9H-purin-6-ylamino)ethyl)phenol (SR1), α-naphthoflavone (CAS 604-59-1), 1,4-dihydroxyanthraquinone, 1,5-dihydroxyanthraquinone, 1,8-dihydroxyanthraquinone, galangin (CAS 548-83-4), resveratrol, 2-methyl-2H-pyrazole-3-carboxylic acid (2-methyl-4-o-tolylazo-phenyl)-amide (CH-223191), N-(2-(3H-Indol-3-yl)ethyl)-9-isopropyl-2-(5-methyl-3-pyridyl)-7H-purin-6-amine (GNF351), 2-(29-amino-39-methoxyphenyl)-oxanaphthalen-4-one (PD98059), (Z)-3-[(2,4-dimethylpyrrol-5-yl)methylidenyl]-2-indolinone (TSU-16), 6,2',4'-trimethoxyflavone (TMF), 3',4'-dimethoxyflavone (DMF), and the like. The AhR antagonist may also be, for example, a compound described as an AhR antagonist in WO 2012/015914. Examples of the expression-suppressing nucleic acid molecule for the AhR include expression-suppressing nucleic acid molecules such as siRNA and miRNA that target the mRNA encoding the AhR. The expression-suppressing nucleic acid molecule for the AhR can be set as appropriate, for example, based on the nucleotide sequence of the AhR mRNA registered in a database. As a specific example, the human AhR mRNA is, for example, a polynucleotide consisting of the nucleotide sequence registered under Accession No. NM_001621 in GenBank. One type of the activity-inhibiting substance for the AhR may be used alone, or two or more types thereof may be used in combination.

In the multinucleation step, the concentration of the AhR inhibitor is not particularly limited, and can be determined as appropriate depending on the type of compound and its effective concentration. Examples of the concentration of the AhR inhibitor include the following concentrations.
SR1: 200 nmol/L or greater and less than 1000 mmol/L
CH-223191: 0.2 µmol/l or greater and less than 4 µmol/l
GNF351: 20 nmol/l or greater and less than 300 nmol/l, or 20 nmol/l or greater and less than 1000 nmol/l
TMF: 2.5 µmol/l or greater and less than 40 µmol/l
DMF: 2.5 µmol/l or greater and less than 40 µmol/l
The ROCK means a Rho-associated coiled-coil forming kinase (ROCK).

In the multinucleation step, the inhibition of the activity of ROCK means, for example, that the activity of ROCK is reduced compared with the case in the absence of the activity-inhibiting substance for the ROCK. The activity of the ROCK can be measured, for example, using the phosphorylation of a substrate by ROCK as an index. The activity of the ROCK can be measured, for example, using a commercially available kit for measuring the activity of ROCK protein, and specific examples thereof include a ROCK activity assay kit (96-Well ROCK Activity Assay Kit, manufactured by Cell Biolabs). In the present disclosure, the inhibition of the activity can also be referred to as, for example, inhibition of the activity, reduction of the activity, suppression of the activation, prevention of the activation, or the like.

Examples of the ROCK inhibitor include a ROCK antagonist, an expression-suppressing nucleic acid molecule capable of suppressing the expression of ROCK, and the like. The ROCK inhibitor includes, for example, (R)-(+)-trans-N-(4-pyridyl)-4-(1-aminoethyl)-cyclohexanecarboxamide (Y-27632), 4-[(1R)-1-aminoethyl]-N-(1H-pyrrolo[2,3-b]pyridin-4-yl)benzamide (Y-39983), fasudil salt (Fasudil (HA1077) hydrochloride), 4-fluoro-5-[[(2S)-hexahydro-2-methyl-1H-1,4-diazepin-1-yl]sulfonyl]-isoquinoline (Ripasudil), 2-(3-(4-((1H-indazol-5-yl)amino)quinazolin-2-yl)phenoxy)-N-isopropylacetamide (SLx-2119), N-[(3-Hydroxyphenyl)methyl]-N'-[4-(4-pyridinyl)-2-thiazolyl]urea dihydrochloride (RKI-1447), 6-Chloro-N4-[3,5-difluoro-4-[(3-methyl-1H-pyrrolo[2,3-b]pyridin-4-yl)oxy]phenyl]-2,4-pyrimidinediamine (TC-S 7001, Azaindole 1), N-[2-[2-(Dimethylamino)ethoxy]-4-(1H-pyrazol-4-yl)phenyl-2,3-dihydro-1,4-benzodioxin-2-carboxamide (SR-3677), Staurosporine, (S)-(+)-2-Methyl-1-[(4-methyl-5-isoquinolinyl)sulfonyl]homopiperazine (H-1152), rac-(2R)-2-(dimethylamino)-N-(1-oxo-1,2-dihydroisoquinolin-6-yl)-2-(thiophen-3-yl)acetamide (AR-12286), N-(1-{[4-(methylsulfanyl)phenyl]methyl}piperidin-3-yl)-1H-indazol-5-amine (INS-117548), and the like. The expression-suppressing nucleic acid molecule for the ROCK includes, for example, expression-suppressing nucleic acid molecules such as siRNA and miRNA that target the mRNA encoding the ROCK. The expression-suppressing nucleic acid molecule for the ROCK can be set as appropriate, for example, based on the nucleotide sequence of the ROCK mRNA registered in a database. As a specific example, the human ROCK1 mRNA is, for example, a polynucleotide consisting of the nucleotide sequence registered under Accession No. NM_005406 in GenBank. Furthermore, the human ROCK2 mRNA is, for example, a polynucleotide consisting of the nucleotide sequence registered under Accession No. NM_004850 in GenBank. One type of the activity-inhibiting substance for the ROCK may be used alone, or two or more types thereof may be used in combination.

In the multinucleation step, the concentration of the ROCK inhibitor is not particularly limited, and can be determined as appropriate depending on the type of compound and its effective concentration. When the activity-inhibiting substance for the ROCK is Y-39983, the concentration of the activity-inhibiting substance for the ROCK is, for example, 1×10⁻⁸ to 1×10⁻³ mol/l, and preferably 1×10⁻⁷ to 1×10⁻⁵ mol/l.

The DYRK means a dual-specificity tyrosine phosphorylation-regulated kinase.

In the inhibition of the activity of DYRK, the target DYRK includes, for example, DYRK1A, DYRK1B, DYRK2, and the like, and DYRK1A is preferable. As an example, the following description is given taking the case of inhibiting the activity of DYRK1A as an example.

In the multinucleation step, the inhibition of the activity of DYRK1A means that the activity of DYRK1A is reduced compared with the case in the absence of the activity-inhibiting substance for the DYRK1A. The activity of the DYRK1A can be measured, for example, using a measurement system employing DYRK1A protein and a substrate, with the phosphorylation of the substrate as an index. For the former, for example, a commercially available kit for measuring the activity of DYRK1A protein can be used, and specific examples thereof include the DYRK1A Kinase Enzyme System (manufactured by Promega, Cat. No. VA7423, VA7424). For the latter, for example, Reference 1 below can be referred to. In the present disclosure, the inhibition of the activity can also be referred to as, for example, inhibition of the activity, reduction of the activity, suppression of the activation, prevention of the activation, or the like.

### Reference 1: Isao Kii et al., "Selective inhibition of the kinase DYRK1A by targeting its folding process", Nat. Commun., 2016, vol. 7, article number 11391

The activity-inhibiting substance for the DYRK1A (a DYRK1A inhibitor) is not particularly limited, and examples thereof include compounds, proteins, nucleic acid molecules, and the like that inhibit the activity or expression of the DYRK1A. As the DYRK1A activity-inhibiting compound, for example, a known DYRK1A activity-inhibiting compound can be used. As specific examples, the DYRK1A activity-inhibiting compound (a DYRK1A antagonist) includes, for example, Harmine (harmine, 7-Methoxy-1-methyl-9H-pyrido[3,4-b]indole, Cas No. 442-51-3), INDY ((1Z)-1-(3-Ethyl-5-hydroxy-2(3H)-benzothiazolylidene)-2-propanone, Pubchem No.: 69538603), GSK626616 ((5Z)-2-[(2,6-Dichlorophenyl)amino]-5-(6-quinoxalinylmethylene)-4(5H)-thiazolone, Cas No. 1025821-33-3), TBB (casein kinase 2 inhibitor 1, 4,5,6,7-tetrabromobenzotriazole, Cas No. 17374-26-4), AZ191 (N-[2-Methoxy-4-(4-methyl-1-piperazinyl)phenyl]-4-(1-methyl-1H-pyrrolo[2,3-c]pyridin-3-yl)-2-pyrimidinamine, Cas No. 1594092-37-1), Mrik-IN-1 (TC-S 7004, N-[2-Chloro-5-[[[(3-chlorophenyl)methyl]amino]carbonyl]phenyl]-7,8-dihydro-2-methoxy-7-oxopyrido[2,3-d]pyrimidine-6-carboxamide, Cas No. 1386979-55-0), EHT5372 (methyl 9-((2,4-dichlorophenyl)amino)thiazolo[5,4-f]quinazoline-2-carbimidate, Cas No. 1425945-60-3), GNF4877 ((R)-1-(3-(3-Amino-6-(2-fluoro-5-isopropoxyphenyl)pyrazine-2-carboxamido)pyridin-4-yl)piperidine-3-carboxylic acid, Cas No. 2041073-22-5), FINDY ((5Z)-5-[[4-Methoxy-3-[2-(trimethylsilyl)ethynyl]phenyl]methylene]-2-thioxo-4-thiazolidinone, Cas No. 1507367-37-4), ML351 (5-(Methylamino)-2-(1-naphthalenyl)-4-oxazolecarbonitrile, Cas No. 847163-28-4), CLK-IN-T3 (N-(6-(3,5-di-tert-butylphenyl)imidazo[1,2-a]pyridin-2-yl)-4-(2-methyl-1-(4-methylpiperazin-1-yl)-1-oxopropan-2-yl)benzamide, Cas No. 2109805-56-1), casein kinase 2 inhibitor IX.IQA (5-Oxo-5,6-dihydroindolo[1,2-a]quinazoline-7-acetic acid, Cas No. 391670-48-7), protein kinase inhibitor 1 ((E)-5-((2-Oxo-6'-(piperazin-1-yl)-1,2-dihydro-[3,3'-bipyridin]-5-yl)methylene)thiazolidine-2,4-dione hydrochloride, Cas No. 1365986-44-2), Leucettine L41 ((5Z)-5-(1,3-benzodioxol-5-ylmethylene)-3,5-dihydro-2-(phenylamino)-4H-imidazol-4-one, Cas No. 1112978-84-3), and the like. The DYRK1A activity-inhibiting compound may, for example, exclude Harmine. As described above, the multinucleation step is carried out in the presence of harmine. Therefore, as the DYRK1A activity-inhibiting compound, it is preferable to use, in addition to the harmine, a compound other than the harmine, and a specific example thereof is AZ191. The activity-inhibiting protein for the DYRK1A includes, for example, a dominant-negative form (DN) protein of DYRK1A. As a specific example, the human DYRK1A DN protein is, for example, a mutant protein in which the 188th lysine (K) is substituted with arginine (R). The expression-suppressing nucleic acid molecule for the DYRK1A includes, for example, expression-suppressing nucleic acid molecules such as siRNA and miRNA that target the mRNA encoding the DYRK1A. The expression-suppressing nucleic acid molecule for the DYRK1A can be set as appropriate, for example, based on the nucleotide sequence of the DYRK1A mRNA registered in a database. As a specific example, the human DYRK1A mRNA is, for example, a polynucleotide consisting of the nucleotide sequence registered under Accession No. NM_001396, NM_101395, NM_130436, NM_130437, NM_130438, NM_001347721, NM_001347722, or NM_001347723 in GenBank. One type of the activity-inhibiting substance for the DYRK1A may be used alone, or two or more types thereof may be used in combination.

The concentration of the activity-inhibiting substance for the DYRK1A is not particularly limited, and can be set as appropriate, for example, depending on the type and effective concentration of the activity-inhibiting substance for the DYRK1A. When the activity-inhibiting substance for the DYRK1A is Harmine, the concentration of the activity-inhibiting substance for the DYRK1A is, for example, 1×10⁻⁸ to 1×10⁻³ mol/l, and preferably 1×10⁻⁶ to 1×10⁻⁴ mol/l. When the activity-inhibiting substance for the DYRK1A is AZ191, the concentration of the activity-inhibiting substance for the DYRK1A is, for example, 1×10⁻⁹ to 1×10⁻³ mol/l, and preferably 1×10⁻⁷ to 1×10⁻⁵ mol/l.

The myosin 2 is a protein that moves on actin with ATPase activity, and means a complex composed of two heavy chains and two light chains attached to each heavy chain (four in total).

In the multinucleation step, the inhibition of the activity of myosin 2 means that the ATPase activity of myosin 2 is reduced compared with the case in the absence of the activity-inhibiting substance for the myosin 2. The activity of the myosin 2 can be measured, for example, using the phosphate released from myosin 2 as an index. The activity of the myosin 2 can be measured, for example, using a commercially available kit for measuring the activity of myosin 2 protein. In the present disclosure, the inhibition of the activity can also be referred to as, for example, inhibition of the activity, reduction of the activity, suppression of the activation, prevention of the activation, or the like.

The myosin 2 inhibitor includes, for example, a myosin 2 antagonist, an expression-suppressing nucleic acid molecule capable of suppressing the expression of myosin 2, a myosin light chain kinase (MLCK) inhibitor, and the like. The myosin 2 inhibitor includes, for example, 3a-Hydroxy-6-methyl-1-phenyl-2,3-dihydropyrrolo[2,3-b]quinolin-4-one (Blebbistatin), CK1122534, Omecamtiv mecarbil (methyl 4-[[2-fluoro-3-[(6-methylpyridin-3-yl)carbamoylamino]phenyl]methyl]piperazine-1-carboxylate), Ammosamides A&B, CTK2018448, Manassantin B ((1R,2R)-1-(1,3-benzodioxol-5-yl)-2-[4-[(2S,3R,4R,5S)-5-[4-[(1R,2R)-1-(3,4-dimethoxyphenyl)-1-hydroxypropan-2-yl]oxy-3-methoxyphenyl]-3,4-dimethyloxolan-2-yl]-2-methoxyphenoxy]propan-1-ol), and the like. The MLCK inhibitor includes, for example, ML7 (Hexahydro-1-[(5-iodo-1-naphthalenyl)sulfonyl]-1H-1,4-diazepine), ML9 (1-(5-chloronaphthalene-1-sulfonyl)-1H-hexahydro-1,4-diazepine), and the like. The expression-suppressing nucleic acid molecule for the myosin 2 includes, for example, expression-suppressing nucleic acid molecules such as siRNA and miRNA that target the mRNA encoding the myosin 2 or myosin light chain kinase. The expression-suppressing nucleic acid molecule for the myosin 2 can be set as appropriate, for example, based on the nucleotide sequence of the mRNA of the heavy chain or light chain constituting myosin 2, or of myosin light chain kinase, registered in a database. As a specific example, the mRNA of the heavy chain constituting human myosin 2 is, for example, a polynucleotide consisting of the nucleotide sequence registered under Accession No. NM_005963, NM_001100112, NM_017534, NM_002470, NM_017533, NM_002147, NM_002471, NM_000257, NM_002472, NM_002473, NM_001256012, NM_002474, NM_001382347, NM_003802, NM_001145809, or NM_014981 in GenBank. The mRNA of the light chain constituting human myosin 2 is, for example, a polynucleotide consisting of the nucleotide sequence registered under Accession No. NM_079420, NM_000432, NM_000258, NM_002476, NM_001363650, NM_021019, NM_021223, NM_006097, NM_138403, or NM_001324458 in GenBank. The mRNA of human myosin light chain kinase is, for example, a polynucleotide consisting of the nucleotide sequence registered under Accession No. NM_001321309, NM_053025, NM_053026, NM_053027, NM_053028, NM_053031, NM_053032, NM_033118, NM_053032, or NM_18249 in GenBank. One type of the activity-inhibiting substance for the myosin 2 may be used alone, or two or more types thereof may be used in combination.

In the multinucleation step, the concentration of the myosin 2 inhibitor is not particularly limited, and can be determined as appropriate depending on the type of compound and its effective concentration. When the activity-inhibiting substance for the myosin 2 is Blebbistatin, the concentration of the activity-inhibiting substance for the myosin 2 is, for example, 1×10⁻⁸ to 1×10⁻³ mol/l, and preferably 1×10⁻⁶ to 1×10⁻⁴ mol/l.

In the multinucleation step, the concentration of the harmine is, for example, 1×10⁻⁸ to 1×10⁻³ mol/l, and preferably 1×10⁻⁶ to 1×10⁻⁴ mol/l.

In the multinucleation step, the inhibition of the activity of AhR, ROCK, DYRK, and/or myosin 2 and the coexistence of harmine may be carried out at the same time or at different times, but are preferably carried out at the same time. That is, the induction from the pre-multinucleated megakaryocyte to the multinucleated megakaryocyte is preferably carried out in a state in which the activity of at least one selected from the group consisting of AhR, ROCK, DYRK, and myosin 2 is inhibited and coexistence with harmine is achieved.

When the pre-multinucleated megakaryocyte is an immortalized megakaryocyte, the multinucleation step can be carried out, for example, as follows. Specifically, the protein expressed from the oncogene, the polycomb gene, and/or the anti-apoptotic gene functions, as described above, as a factor that promotes the proliferation of the pre-multinucleated megakaryocyte in the immortalized megakaryocyte. Accordingly, when the immortalized megakaryocyte is used, the immortalized megakaryocyte induces a multinucleated megakaryocyte, for example, by maintaining the forced expression of the oncogene, the polycomb gene, and/or the anti-apoptotic gene, inhibiting the activity of at least one selected from the group consisting of AhR, ROCK, DYRK, and myosin 2, and culturing in the presence of harmine. In this case, in the multinucleation step, during the culture of the immortalized megakaryocyte, the medium containing the immortalized megakaryocyte is replaced with a medium containing harmine and at least one selected from the group consisting of the AhR inhibitor, the ROCK inhibitor, the DYRK inhibitor, and the myosin 2 inhibitor, or harmine and at least one selected from the group consisting of the AhR inhibitor, the ROCK inhibitor, the DYRK inhibitor, and the myosin 2 inhibitor are added to the medium containing the immortalized megakaryocyte. The addition or replacement may be carried out, for example, once, or two or more times.

The medium in the multinucleation step is not particularly limited, and examples thereof include known media suitable for producing the megakaryocyte and media equivalent thereto. As a specific example, the medium can be prepared, for example, using a medium used for culturing animal cells as a basal medium. Examples of the basal medium include a single medium such as IMDM medium, Medium 199 medium, Eagle's Minimum Essential Medium (EMEM) medium, αMEM medium, Dulbecco's modified Eagle's Medium (DMEM), Ham's F12 medium, RPMI1640 medium, Fischer's medium, and Neurobasal^{®} Medium (manufactured by Thermo Fisher Scientific), or a mixed medium thereof. The medium may contain, for example, serum or plasma, or may be a serum-free medium not containing these, but a serum-free medium is preferable. By using a serum-free medium as the medium of the multinucleation step, for example, the degree of multinucleation from the pre-multinucleated megakaryocyte to the multinucleated megakaryocyte can be improved. The serum-free medium includes, for example, StemSpan ACF (manufactured by StemCell Technologies, Cat. No.: 9855), Stemline 2 (manufactured by Sigma Aldrich, Cat. No.: S0192), and the like. The origin of the serum and plasma is preferably the same as the origin of the megakaryocyte. As a specific example, when the megakaryocyte is derived from a human, the serum and plasma are each preferably derived from a human. By making the origin of the megakaryocyte and the serum the same, for example, the multinucleation of the megakaryocyte can be further promoted in the multinucleation step.

The medium may contain, for example, other components. The other components are not particularly limited, and examples thereof include albumin, insulin, transferrin, selenium, fatty acids, trace elements, 2-mercaptoethanol, thiolglycerol, monothioglycerol (MTG), lipids, amino acids (for example, L-glutamine), ascorbic acid, heparin, non-essential amino acids, vitamins, growth factors, low-molecular-weight compounds, antibiotics, antioxidants, pyruvic acid, buffers, inorganic salts, cytokines, and the like. The other components may be, for example, one type or two or more types. The cytokine is, for example, a substance that promotes the differentiation of blood cells, and specific examples thereof include vascular endothelial growth factor (VEGF), thrombopoietin (TPO), various TPO-like agents, Stem Cell Factor (SCF), ITS (insulin-transferrin-selenite) supplement, ADAM inhibitors, FLT inhibitors, WNT inhibitors, and the like. The medium is, for example, preferably an IMDM medium containing serum, insulin, transferrin, serine, thiolglycerol, ascorbic acid, and TPO. The medium may further contain, for example, SCF, and may further contain heparin. The concentration of the other components is not particularly limited. The concentration of the TPO is, for example, approximately 10 ng/ml to approximately 200 ng/ml, or approximately 50 ng/ml to approximately 100 ng/ml. The concentration of the SCF is, for example, approximately 10 ng/ml to approximately 200 ng/ml, or approximately 50 ng/ml. The concentration of the heparin is, for example, approximately 10 U/ml to approximately 100 U/ml, or approximately 25 U/ml. The medium may further contain, for example, a phorbol ester (for example, phorbol-12-myristate-13-acetate; PMA).

In the multinucleation step, the cell density of the pre-multinucleated megakaryocyte at the start is not particularly limited. The lower limit of the cell density is, for example, 1×10⁵ cells/ml, 2×10⁵ cells/ml, 3×10⁵ cells/ml, or 4×10⁵ cells/ml. The upper limit of the cell density is not particularly limited, and is, for example, 4×10⁵ cells/ml, 6×10⁵ cells/ml, 8×10⁵ cells/ml, 1×10⁷ cells/ml, 5×10⁷ cells/ml, or 1×10⁸ cells/ml. The range of the cell density is, for example, 1×10⁵ cells/ml to 1×10⁸ cells/ml, 1×10⁵ cells/ml to 5×10⁷ cells/ml, 1×10⁵ cells/ml to 1×10⁷ cells/ml, 1×10⁵ cells/ml to 8×10⁵ cells/ml, 1×10⁵ cells/ml to 5×10⁵ cells/ml, 2×10⁵ cells/ml to 8×10⁵ cells/ml, 3×10⁵ cells/ml to 6×10⁵ cells/ml, or 4×10⁵ cells/ml to 6×10⁵ cells/ml, and, because multinucleation can be promoted or the production of platelets can be improved, is preferably 1×10⁵ cells/ml to 8×10⁵ cells/ml or 1×10⁵ cells/ml to 5×10⁵ cells/ml. The cell density can be calculated, for example, by dividing the number of the pre-multinucleated megakaryocytes by the volume of the medium in which the pre-multinucleated megakaryocytes are suspended.

The culture conditions in the multinucleation step are not particularly limited, and the ordinary culture conditions for the megakaryocyte can be employed. As specific examples, the culture temperature is, for example, approximately 35°C to approximately 42°C, approximately 36°C to approximately 40°C, or approximately 37°C to approximately 39°C. The CO₂ concentration is, for example, approximately 5% to approximately 15%. The O₂ concentration is, for example, approximately 15% to approximately 25%, or approximately 20%.

The culture period of the multinucleation step can be set, for example, as the period until the pre-multinucleated megakaryocyte is multinucleated. The culture period of the multinucleation step may be set as appropriate depending on the proportion of multinucleated megakaryocytes among the megakaryocytes during culture. The culture period of the multinucleation step is preferably set, for example, to a period in which the proportion of multinucleated megakaryocytes of 8N or more among the megakaryocytes during culture becomes 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 35% or more, 40% or more, 45% or more, 50% or more, 55% or more, or 60% or more. As a specific example, the culture period of the multinucleation step is, for example, 0 to 20 days, or 1 to 15 days. The proportion of multinucleated megakaryocytes among the megakaryocytes can be calculated, for example, by extracting a portion of the megakaryocytes during culture.

In the multinucleation step, the culture of the pre-multinucleated megakaryocyte may be carried out, for example, on feeder cells, or may be carried out without feeder cells. The pre-multinucleated megakaryocyte can be, for example, cultured in suspension, and therefore can be cultured without the feeder cells. The feeder cell means a cell that is co-cultured with a cell to be proliferated or differentiated (target cell in order to prepare the environment necessary for culturing the target cell. The feeder cell may be any cell distinguishable from the target cell, and may be a cell derived from the same species as the target cell or a cell derived from a different species. The feeder cell may be, for example, a cell treated so as not to proliferate by antibiotics, anticancer agents, γ-ray irradiation, or the like.

The multinucleated megakaryocyte after the multinucleation step may be composed, for example, only of multinucleated megakaryocytes, or may include other cells. In the latter case, the multinucleated megakaryocyte after the multinucleation step can also be referred to as, for example, a cell population containing multinucleated megakaryocytes. The multinucleated megakaryocyte may be, for example, a multinucleated megakaryocyte of 8N or more, a multinucleated megakaryocyte of 16N or more, or a multinucleated megakaryocyte of 32N or more. The multinucleated megakaryocytes after the multinucleation step include, for example, the multinucleated megakaryocyte of 8N or more, the multinucleated megakaryocyte of 16N or more, and/or the multinucleated megakaryocyte of 32N or more.

When the multinucleated megakaryocytes include multinucleated megakaryocytes having a ploidy of 8N or more, in the cell population, the lower limit value of the proportion (cell number) of the multinucleated megakaryocytes of 8N or more is, for example, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 35% or more, 40% or more, 45% or more, 50% or more, 55% or more, or 60% or more. In the cell population, the upper limit value of the proportion (cell number) of the multinucleated megakaryocytes of 8N or more is, for example, 90% or less, 85% or less, 80% or less, 75% or less, 70% or less, 65% or less, 60% or less, 59% or less, 58% or less, 57% or less, 56% or less, 55% or less, 54% or less, 53% or less, 52% or less, 51% or less, 50% or less, 49% or less, 48% or less, 47% or less, or 46% or less. The proportion (cell number) of the multinucleated megakaryocytes of 8N or more is, for example, 15% to 90%, 20% to 90%, 25% to 90%, 30% to 90%, 35% to 90%, 40% to 90%, 45% to 90%, 50% to 90%, 55% to 90%, 60% to 90%, 15% to 85%, 15% to 80%, 15% to 75%, 15% to 70%, 15% to 65%, 16% to 60%, 17% to 59%, 18% to 58%, 19% to 57%, 20% to 56%, 21% to 55%, 22% to 54%, 23% to 54%, 24% to 53%, 25% to 51%, 26% to 50%, 27% to 49%, 28% to 48%, 29% to 47%, or 30% to 46%.

When the multinucleated megakaryocytes include multinucleated megakaryocytes having a ploidy of 16N or more, in the cell population, the lower limit value of the proportion (cell number) of the multinucleated megakaryocytes of 16N or more is, for example, 15% or more, 16% or more, 17% or more, 18% or more, 19% or more, 20% or more, 21% or more, 22% or more, 23% or more, 24% or more, or 25% or more. In the cell population, the upper limit value of the proportion (cell number) of the multinucleated megakaryocytes of 16N or more is, for example, 90% or less, 85% or less, 80% or less, 75% or less, 70% or less, 65% or less, 60% or less, 55% or less, 54% or less, 53% or less, 52% or less, 51% or less, 50% or less, 49% or less, 48% or less, 47% or less, 46% or less, 45% or less, 44% or less, or 43% or less. The proportion (cell number) of the multinucleated megakaryocytes of 16N or more is, for example, 15% to 90%, 15% to 85%, 15% to 80%, 15% to 75%, 15% to 70%, 15% to 65%, 15% to 60%, 16% to 55%, 17% to 54%, 18% to 53%, 19% to 52%, 20% to 90%, 20% to 70%, 20% to 51%, 21% to 50%, 22% to 49%, 23% to 48%, 24% to 47%, 25% to 46%, 25% to 45%, 25% to 44%, or 25% to 43%.

When the multinucleated megakaryocytes include multinucleated megakaryocytes having a ploidy of 32N or more, in the cell population, the lower limit value of the proportion (cell number) of the multinucleated megakaryocytes of 32N or more is, for example, 5% or more, 6% or more, 7% or more, 8% or more, 9% or more, 10% or more, 11% or more, 12% or more, 13% or more, 14% or more, or 15% or more. In the cell population, the upper limit value of the proportion (cell number) of the multinucleated megakaryocytes of 32N or more is, for example, 50% or less, 49% or less, 48% or less, 47% or less, 46% or less, 45% or less, 44% or less, 43% or less, 42% or less, 41% or less, 40% or less, 39% or less, 38% or less, 37% or less, 36% or less, 35% or less, 34% or less, 33% or less, 32% or less, 31% or less, or 30% or less. The proportion (cell number) of the multinucleated megakaryocytes of 32N or more is, for example, 5% to 50%, 5% to 49%, 5% to 48%, 5% to 47%, 5% to 46%, 5% to 45%, 5% to 44%, 5% to 43%, 5% to 42%, 5% to 41%, 5% to 40%, 6% to 39%, 7% to 38%, 8% to 37%, 9% to 36%, 10% to 35%, 11% to 34%, 12% to 33%, 13% to 32%, 14% to 31%, 15% to 31%, or 15% to 30%.

The pre-multinucleated megakaryocyte can be induced, for example, from a cell more undifferentiated than the megakaryocyte (also referred to as a "progenitor cell of the pre-multinucleated megakaryocyte"). Therefore, the production method may include, for example, prior to the multinucleation step, a megakaryocyte induction step of inducing a pre-multinucleated megakaryocyte from a cell more undifferentiated than the megakaryocyte. For the medium, culture conditions, and the like used in the megakaryocyte induction step, for example, the description of the above-described multinucleation step can be applied.

The "cell more undifferentiated than the megakaryocyte" means a cell having the ability to differentiate into the megakaryocyte. As specific examples, the cell more undifferentiated than the megakaryocyte includes, for example, a hematopoietic stem cell, a hematopoietic progenitor cell, a CD34-positive cell, a megakaryocyte-erythroid progenitor (MEP), a megakaryocyte progenitor cell, and the like. The cell more undifferentiated than the megakaryocyte may be isolated, for example, from bone marrow, umbilical cord blood, peripheral blood, or the like, or may be induced from a pluripotent cell or pluripotent stem cell such as an embryonic stem cell (ES cell), an induced pluripotent stem cell (iPS cell), a nuclear transfer ES cell (ntES cell), a germline stem cell, a somatic stem cell, or an embryonal carcinoma cell.

The method for inducing the megakaryocyte is not particularly limited, and can be carried out by a known induction method. As specific examples, the method for inducing the megakaryocyte includes, for example, the methods described in No. WO 2011/034073, WO 2012/157586, and the like. As a specific example, in the megakaryocyte induction step, for example, an oncogene and a polycomb gene may be forcibly expressed in the cell more undifferentiated than the megakaryocyte. Thereby, in the megakaryocyte induction step, for example, an infinitely proliferating immortalized megakaryocyte corresponding to a pre-multinucleated megakaryocyte can be obtained. Furthermore, for example, by halting the forced expression in the immortalized megakaryocyte, the immortalized megakaryocyte is induced into a multinucleated megakaryocyte, and, furthermore, an enlarged megakaryocyte can be induced. Therefore, the immortalized megakaryocyte corresponds to a pre-multinucleated megakaryocyte. Furthermore, in the megakaryocyte induction step, for example, an anti-apoptotic gene may be forcibly expressed in the megakaryocyte progenitor cell. Thereby, in the megakaryocyte induction step, the immortalized megakaryocyte can be obtained. Furthermore, in the enlargement step described below, for example, by halting the forced expression in the immortalized megakaryocyte, the immortalized megakaryocyte matures and is induced into the enlarged megakaryocyte.

In the megakaryocyte induction step, for example, the oncogene, the polycomb gene, and the anti-apoptotic gene may be forcibly expressed. In this case, the forced expression of the oncogene, the polycomb gene, and the anti-apoptotic gene may be carried out simultaneously or separately. As specific examples, in the megakaryocyte induction step, after the oncogene and the polycomb gene are forcibly expressed, the forced expression may be halted and then the anti-apoptotic gene may be forcibly expressed; the oncogene, the polycomb gene, and the anti-apoptotic gene may be forcibly expressed; or the oncogene and the polycomb gene may be forcibly expressed and, furthermore, the anti-apoptotic gene may be expressed. Thereby, in the megakaryocyte induction step, the immortalized megakaryocyte can be obtained. Furthermore, in the enlargement step described below, for example, by halting the forced expression in the immortalized megakaryocyte, the immortalized megakaryocyte matures and is induced into the enlarged megakaryocyte.

The megakaryocyte induction step preferably includes, for example, because the introduction efficiency of each gene can be improved, a first expression step of forcibly expressing an oncogene and a polycomb gene in the cell more undifferentiated than the megakaryocyte, a second expression step of forcibly expressing an anti-apoptotic gene such as the *BCL-XL* gene in the undifferentiated cell, and a halting step of halting all of the forced expression. As described above, by halting the forced expression, for example, a multinucleated megakaryocyte is induced from the immortalized megakaryocyte, and, furthermore, the enlarged megakaryocyte is induced. Therefore, the halting step can also be referred to as the enlargement step described below.

The forced expression of each gene and the halting of the forced expression can be carried out by a known method such as the methods described in WO 2011/034073, WO 2012/157586, WO 2014/123242, or Reference 2 below, or by a method equivalent thereto. As a specific example, the forced expression of each gene and the halting of the forced expression can be carried out, for example, using a drug-responsive gene expression induction system. Examples of the gene expression induction system include the Tet-on^{®} system, the Tet-off^{®} system, and the like. When the Tet-on system is used, for example, in the step of forcibly expressing, culture is carried out in the presence of a drug that induces gene expression, such as tetracycline or doxycycline, and, in the step of halting the forced expression, the culture is carried out in the absence of the drug.

### Reference 2: Nakamura S et al., "Expandable megakaryocyte cell lines enable clinically applicable generation of platelets from human induced pluripotent stem cells.", Cell Stem Cell, 2014, vol. 14, No. 4, pages 535-548

In the present disclosure, an "oncogene" means a gene capable of inducing the canceration of a cell *in vivo*, and examples thereof include MYC family genes such as c-MYC, N-MYC, and L-MYC; and protein kinase family genes such as SRC family genes, RAS family genes, RAF family genes, c-kit (CD117), PDGFR (platelet-derived growth factor receptor), and Abl (Abelson murine leukemia viral oncogene homolog).

In the present disclosure, a "polycomb gene" means a gene known to negatively regulate CDKN2a (cyclin-dependent kinase inhibitor 2A, INK4a/ARF) and to function to avoid cellular senescence (References 3 to 5 below). As specific examples, the polycomb gene includes, for example, BMI1 (Polycomb complex protein BMI-1, polycomb group RING finger protein 4 (PCGF4), RING finger protein 51 (RNF51)), Mel18 (Polycomb group RING finger protein 2), Ring (Ring Finger Protein) 1a/b, Phc (Polyhomeotic Homolog) 1/2/3, Cbx (Chromobox) 2/4/6/7/8, Ezh2 (Enhancer Of Zeste 2 Polycomb Repressive Complex 2 Subunit), Eed (Embryonic Ectoderm Development), Suz12 (SUZ12 Polycomb Repressive Complex 2 Subunit), HADC (Histone deacetylases), Dnmt (DNA (cytosine-5)-methyltransferase) 1/3a/3b, and the like.

### Reference 3: Hideyuki Oguro et al., "Regulation of stem cell senescence by polycomb group protein complexes", Regenerative Medicine, 2007, vol. 6, No. 4, pages 26-32

### Reference 4: Jesus Gil et al., "Regulation of the INK4b-ARF-INK4a tumour suppressor locus: all for one or one for all", Nature Reviews Molecular Cell Biology, 2007, vol. 7, pages 667-677

### Reference 5: Soo-Hyun Kim et al., "Absence of p16INK4a and truncation of ARF tumor suppressors in chickens", PNAS, 2003, vol. 100, No. 1, pages 211-216

In the present disclosure, an "anti-apoptotic gene" means a gene having the function of being able to suppress the apoptosis of a cell, and examples thereof include BCL2 (B-cell lymphoma 2), BCL-XL (B-cell lymphoma-extra large), Survivin (Baculoviral IAP Repeat Containing 5), MCL1 (BCL2 Family Apoptosis Regulator), and the like.

The production method may include a proliferation step of proliferating the pre-multinucleated megakaryocyte in the presence of a factor that promotes the proliferation of the pre-multinucleated megakaryocyte. In this case, in the proliferation step, the pre-multinucleated megakaryocyte is proliferated without inhibiting the activity of AhR, ROCK, DYRK, and myosin 2, and in the absence of harmine. Thereby, in the proliferation step, the pre-multinucleated megakaryocyte can be proliferated while maintaining the state before multinucleation. Specifically, in the proliferation step, the pre-multinucleated megakaryocyte is cultured in the presence of a factor that promotes the proliferation of the pre-multinucleated megakaryocyte, and the pre-multinucleated megakaryocyte is proliferated. The factor that promotes the proliferation of the pre-multinucleated megakaryocyte may be present in the medium, or may be present within the pre-multinucleated megakaryocyte. Furthermore, in the proliferation step, by not adding, to the medium, substances that inhibit the activity of AhR, ROCK, DYRK, and myosin 2, and harmine, the proliferation can be carried out without inhibiting the activity of AhR, ROCK, DYRK, and myosin 2, and in the absence of harmine. For the medium, culture conditions, and the like used in the proliferation step, for example, the description of the above-described multinucleation step can be applied.

When the production method includes the proliferation step, the multinucleation step is carried out after the proliferation step. The growth factor for the pre-multinucleated megakaryocyte includes, for example, stem cell factor (SCF), thrombopoietin (TPO), and the like.

The concentration of the growth factor for the pre-multinucleated megakaryocyte is not particularly limited, and can be set as appropriate, for example, depending on the type and effective concentration of the growth factor. When the growth factor is SCF or TPO, the concentration of the growth factor is, for example, 0.1 to 1000 ng/ml, or 1 to 200 ng/ml.

The culture period of the proliferation step can be set, for example, as the period until a desired number of pre-multinucleated megakaryocytes are obtained. Specifically, the culture period of the proliferation step is, for example, 1 day or more, and specific examples thereof are 1 to 20 days, 1 to 15 days, 1 to 10 days, 1 to 5 days, 2 to 5 days, or 2 to 4 days.

When the immortalized megakaryocyte is used as the pre-multinucleated megakaryocyte, the immortalized megakaryocyte proliferates while maintaining the state before multinucleation when the oncogene and the polycomb gene, or the oncogene, the polycomb gene, and the anti-apoptotic gene, are expressed. Therefore, when the immortalized megakaryocyte is used, the first expression step and/or the second expression step can be referred to as the proliferation step. Furthermore, the protein encoded by the oncogene, the polycomb gene, and/or the anti-apoptotic gene, that is, the protein expressed from the oncogene, the polycomb gene, and/or the anti-apoptotic gene, can also be referred to as a factor that promotes the proliferation of the pre-multinucleated megakaryocyte. When the immortalized megakaryocyte is used as the pre-multinucleated megakaryocyte, for the proliferation step, the description of the megakaryocyte induction step relating to the immortalized megakaryocyte can be applied.

In the enlargement step, the multinucleated megakaryocyte is matured to induce an enlarged megakaryocyte. Specifically, the enlargement step can be carried out, for example, by culturing the multinucleated megakaryocyte in the presence of a medium. The culture of the multinucleated megakaryocyte may be carried out, for example, on feeder cells, or may be carried out without feeder cells. When the multinucleated megakaryocyte is derived from an immortalized megakaryocyte, the enlargement step can be carried out, for example, by suppressing the expression of the oncogene and the polycomb gene, or the oncogene, the polycomb gene, and the anti-apoptotic gene, in the multinucleated megakaryocyte.

In the enlargement step, the period for inducing the enlarged megakaryocyte from the multinucleated megakaryocyte is not particularly limited, and is, for example, 1 to 10 days, 1 to 5 days, or 2 to 3 days. In the enlargement step, for example, by making the induction period relatively long, the content of the secretome, the extracellular vesicles, and/or the factors in the obtained cultured medium can be increased.

In the enlargement step, for example, the pre-multinucleated megakaryocyte may be proliferated by inhibiting the activity of AhR, ROCK, DYRK, and myosin 2, and in the presence of harmine. In this case, in the enlargement step, by adding, to the medium, substances that inhibit the activity of AhR, ROCK, DYRK, and myosin 2, and harmine, the enlargement can be carried out by inhibiting the activity of AhR, ROCK, DYRK, and myosin 2, and in the presence of harmine. The enlargement step may be carried out, for example, without inhibiting the activity of AhR, ROCK, DYRK, and myosin 2, and in the absence of harmine. In this case, the enlargement step can be carried out, for example, by replacing, after the multinucleation step, the medium of the multinucleated megakaryocyte with a medium not containing the substances that inhibit the activity of AhR, ROCK, DYRK, and myosin 2, and harmine. For the medium, culture conditions, and the like used in the enlargement step, for example, the description of the above-described multinucleation step can be applied.

In the enlargement step, the cell density of the multinucleated megakaryocyte at the start of the induction of the enlarged megakaryocyte is not particularly limited. The lower limit of the cell density is, for example, 1×10⁵ cells/ml, 2×10⁵ cells/ml, 3×10⁵ cells/ml, or 4×10⁵ cells/ml. The upper limit of the cell density is not particularly limited, and is, for example, 4×10⁵ cells/ml, 6×10⁵ cells/ml, 8×10⁵ cells/ml, 1×10⁶ cells/ml, or 1×10⁷ cells/ml. The range of the cell density is, for example, 1×10⁵ cells/ml to 8×10⁵ cells/ml, 1×10⁵ cells/ml to 1×10⁶ cells/ml, 1×10⁵ cells/ml to 1×10⁷ cells/ml, 2×10⁵ cells/ml to 8×10⁵ cells/ml, 3×10⁵ cells/ml to 6×10⁵ cells/ml, or 4×10⁵ cells/ml to 6×10⁵ cells/ml. The cell density can be calculated, for example, by dividing the number of the multinucleated megakaryocytes by the volume of the medium in which the multinucleated megakaryocytes are suspended.

In this manner, in the present disclosure, the enlarged megakaryocyte can be induced from the megakaryocyte. The enlarged megakaryocyte secretes the secretome and/or the extracellular vesicles into the medium, for example, by being cultured in the medium. Therefore, the culture medium (cultured medium) during the enlargement step, the cultured medium after the enlargement step, or the cultured medium of the enlarged megakaryocyte cultured after the enlargement step (hereinafter also referred to as the "cultured medium of the multinucleated megakaryocyte") contains, for example, the secretome and/or the extracellular vesicles. The bone-forming composition of the present disclosure may contain, for example, as the secretome and/or the extracellular vesicles, the cultured medium of the enlarged megakaryocyte.

During or after the enlargement step, the present disclosure may include, for example, a stimulation step of stimulating the enlarged megakaryocyte in order to increase the amount of the secretome and/or the extracellular vesicles released into the cultured medium. Examples of the stimulation step include optical stimulation such as X-ray irradiation; physical stimulation such as preparation using glass beads; chemical stimulation such as collagen, ADP, and TRAP6; and the like.

After the enlargement step, a concentration step of concentrating the cultured medium may be included. The concentration may be carried out using a concentration member, or may be carried out by centrifugation or the like. Examples of the concentration member include a membrane filter, an ultrafiltration membrane, a hollow-fiber membrane, and the like. The pore size of the concentration member is, for example, a pore size capable of capturing the secretome and/or the extracellular vesicles. As a specific example, the pore size is, for example, 50 to 100 nm. The concentration step is preferably carried out after the stimulation step.

The bone-forming composition of the present disclosure may contain, for example, the concentrate of the cultured medium after the concentration step. The concentrate is, for example, a liquid or a solid.

The present disclosure may include, for example, an isolation step of isolating the extracellular vesicles from the cultured medium after the enlargement step. The isolation may be carried out, for example, using ultracentrifugation, density gradient ultracentrifugation, size exclusion chromatography, affinity purification, co-precipitation, or the like, or may be carried out by combining these.

The bone-forming composition of the present disclosure may contain, for example, the extracellular vesicles isolated after the isolation step. The term "isolation" means identifying and separating, and being taken out from a natural (nature) state.

The bone-forming composition of the present disclosure may have, for example, cell proliferation-promoting activity. When the bone-forming composition of the present disclosure has cell proliferation-promoting activity, the bone-forming composition of the present disclosure can also be referred to as, for example, a "composition for promoting cell proliferation." In the cell proliferation-promoting activity, the cell is not particularly limited, and examples thereof include a mesenchymal stem cell and the like. The mesenchymal stem cell is a cell having self-renewal ability and the ability to differentiate into bone, cartilage, and adipocytes. The mesenchymal stem cell can be identified by cell surface markers. The mesenchymal stem cell is, for example, positive for CD73, CD90, and CD105, and negative for CD14, CD34, and CD45. The bone-forming composition of the present disclosure may be used, for example, in combination with a mesenchymal stem cell.

The cell proliferation-promoting activity may be such that, for example, the proliferative capacity of the cell is improved compared with a control group that is the same except that the bone-forming composition of the present disclosure is not added, and, for example, the proliferative capacity of the cell may be reduced from the start. In this case, the "proliferation-promoting activity" can also be referred to as, for example, suppression of the reduction in proliferative activity. As a specific example, the mesenchymal stem cell decreases in proliferative activity with each passage. According to the bone-forming composition of the present disclosure, since the reduction in proliferative activity of the mesenchymal stem cell can be suppressed, for example, the bone-forming composition of the present disclosure can be said to exhibit proliferation-promoting activity. The cell proliferation-promoting activity can be measured, for example, under culture conditions in which the target cell proliferates. The culture conditions can be set as appropriate, for example, depending on the type of the cell.

The activity of promoting differentiation into osteoblasts may be such that, for example, the ability to differentiate into osteoblasts is improved compared with a control group that is the same except that the bone-forming composition of the present disclosure is not added. The improvement in differentiation ability may mean either that the rate of differentiation into osteoblasts becomes faster, or that the proportion of cells that differentiate into osteoblasts is improved. For the differentiation into osteoblasts, for example, Japanese Unexamined Patent Application Publication No. 2012-120529 can be referred to.

The bone-forming composition of the present disclosure may be used, for example, *in vitro* or *in vivo*.

When the bone-forming composition of the present disclosure is used *in vitro*, examples of the administration subject include cells, tissues, organs, and the like, and examples of the cells include cells collected from a living body, cultured cells, and the like.

When the bone-forming composition of the present disclosure is used *in vivo*, the administration subject includes, for example, humans and non-human animals excluding humans. The non-human animal includes, for example, mice, rats, rabbits, dogs, sheep, horses, cats, goats, monkeys, guinea pigs, and the like.

The conditions of use (administration conditions) of the bone-forming composition of the present disclosure are not particularly limited, and, for example, the mode of administration, the timing of administration, the dose, and the like can be set as appropriate depending on the type of administration subject and the like.

When the bone-forming composition of the present disclosure is used *in vitro*, the bone-forming composition of the present disclosure can be used, for example, by adding it to the medium of a target progenitor cell. The bone-forming composition of the present disclosure may be added, for example, to a maintenance medium used when maintaining the progenitor cell.

When the bone-forming composition of the present disclosure is used *in vivo*, it can be determined as appropriate, for example, depending on the type of administration subject, the symptoms, the age, the administration method, and the like.

The mode of administration of the bone-forming composition of the present disclosure is not particularly limited. When the bone-forming composition of the present disclosure is administered *in vivo*, administration may be oral administration or parenteral administration. The parenteral administration includes, for example, intravenous injection (intravenous administration), intramuscular injection (intramuscular administration), transdermal administration, subcutaneous administration, intradermal administration, enteral administration, rectal administration, vaginal administration, nasal administration, pulmonary administration, intraperitoneal administration, topical administration, and the like.

The dosage form of the bone-forming composition of the present disclosure is not particularly limited, and can be determined as appropriate, for example, depending on the mode of administration. The dosage form includes, for example, a liquid form and a solid form.

The bone-forming composition of the present disclosure may contain, for example, an additive as necessary. The additive is preferably a pharmaceutically acceptable additive or a pharmaceutically acceptable carrier.

The bone-forming composition of the present disclosure may contain, for example, a lysate of the enlarged megakaryocyte. The lysate can also be referred to as, for example, a lysate, and can be prepared by physically or chemically disrupting the enlarged megakaryocyte. The lysate is, for example, a liquid or a solid.

It is presumed that the bone-forming composition of the present disclosure can be suitably used, for example, for jawbone defects due to jawbone tumors, cysts, osteonecrosis, trauma, or skeletal dysplasias (ectodermal dysplasia, cleft lip and palate, etc.); alveolar bone defects such as alveolar bone defects associated with periodontal disease, peri-implantitis, or the like, and alveolar bone defects due to caries or trauma; osteoporosis such as primary osteoporosis (postmenopausal osteoporosis, senile osteoporosis, etc.) and secondary osteoporosis (osteogenesis imperfecta, etc.); refractory fractures due to hyperthyroidism, Cushing's syndrome, hypogonadism), trauma, and the like; rib joining by thoracotomy; bone defects due to craniotomy; systemic bone defects due to tumor resection or cyst removal; and the like.

### <Bone-Forming Kit>

The bone-forming kit of the present disclosure comprises a bone-forming composition and a scaffold material for bone formation, wherein the bone-forming composition is the bone-forming composition of the present disclosure. The kit of the present disclosure can readily induce bone formation by combining the bone-forming composition of the present disclosure with the scaffold material. For the kit of the present disclosure, the description of the bone-forming composition of the present disclosure can be applied.

In the kit of the present disclosure, as the scaffold material, for example, a scaffold material used for bone or cartilage regeneration can be used. The scaffold material can also be referred to as, for example, a material to which osteocytes or osteoblasts, or progenitor cells thereof, can attach, proliferate, and/or differentiate. The scaffold material is, for example, a structure having a three-dimensional hollow structure or porous structure, and specific examples thereof include a porous body, a porous support, a porous three-dimensional structure, a gel (hydrogel), a sponge structure, and the like.

The scaffold material is preferably, for example, biocompatible and/or biodegradable, because it is placed in a living body together with the composition of the present disclosure. The "biocompatibility" means, for example, having affinity with tissues and/or organs in a living body and not causing foreign body reactions, rejection reactions, or the like. The "biodegradability" means, for example, that the constituent components thereof are degradable in a living body.

The constituent components of the scaffold material include, for example, proteins such as collagen, gelatin, albumin, keratin, fibrin, and fibroin; polysaccharides such as agarose, dextran, carboxymethyl cellulose, xanthan gum, chitosan, chondroitin sulfate, heparin, hyaluronic acid, and alginic acid; polyglycolic acid (PGA), polylactic acid (PLA), a copolymer of polyglycolic acid and polylactic acid, polyhydroxybutyrate, polydioxanone, polyethylene glycol (PEG), polycaprolactone, polybutylene succinate, calcium phosphate (for example, beta-tricalcium phosphate), calcium carbonate, hydroxyapatite, polyether ketone, polyether ether ketone, and the like. One type of the constituent component may be used alone, or a plurality of types may be used in combination.

The scaffold material can be prepared, for example, by crosslinking or compositing the constituent components of the scaffold material.

The scaffold material includes, for example, NEOBONE (made of hydroxyapatite, manufactured by Aimdic MMT), APACERAM (registered trademark, made of hydroxyapatite, manufactured by HOYA Corporation), Bonarc (made of collagen/octacalcium phosphate, manufactured by Toyobo Co., Ltd.), OSferion (made of β-TCP, manufactured by Olympus Terumo Biomaterials Corp.), Cytrans Granules (made of carbonate apatite, manufactured by GC Corporation), ReFit (made of collagen/apatite, manufactured by HOYA Corporation), and the like.

In the kit of the present disclosure, the bone-forming composition and the scaffold material may be present independently or may be present integrally. In the latter case, the kit of the present disclosure can also be referred to as, for example, a bone-forming scaffold material. When the bone-forming composition and the scaffold material are integral, the bone-forming composition may, for example, be adsorbed on the surface of the scaffold material, or may be contained within the scaffold material.

The kit of the present disclosure may contain, for example, in addition to the bone-forming composition, a physiologically active substance. The physiologically active substance is, for example, a substance that promotes bone formation or the differentiation of osteoblasts or osteocytes. The physiologically active substance includes, for example, vascular endothelial growth factor (VEGF), platelet-derived growth factor (PDGF), epidermal growth factor (EGF), fibroblast growth factor (FGF), hepatocyte growth factor (HGF), insulin-like growth factor (IGF), brain-derived neurotrophic factor (BDNF), growth differentiation factor-5 (GDF5), erythropoietin (EPO), transforming growth factor (TGF), bone morphogenetic protein (BMP), and the like. One type of the physiologically active substance may be used alone, or a plurality of types may be used in combination.

The kit of the present disclosure can be used, for example, by transplanting (embedding, implanting) the bone-forming composition and the scaffold material at a desired location where bone formation is to be induced in the administration subject. Specifically, in the kit of the present disclosure, when the composition and the scaffold material are independent, the kit can be used by impregnating the scaffold material with the bone-forming composition and then transplanting the scaffold material. Furthermore, in the kit of the present disclosure, when the bone-forming composition and the scaffold material are independent, the kit may be used by transplanting the scaffold material and then introducing the bone-forming composition into the transplantation site. Furthermore, in the kit of the present disclosure, when the bone-forming composition and the scaffold material are integral, the kit can be used by transplanting the scaffold material.

### <Bone-Forming Method>

The bone-forming method of the present disclosure uses the bone-forming composition of the present disclosure. According to the bone-forming method of the present disclosure, for example, bone formation can be induced. For the bone-forming method of the present disclosure, the descriptions of the bone-forming composition and the bone-forming kit of the present disclosure can be applied.

The bone-forming method of the present disclosure includes, for example, an administration step of administering the bone-forming composition of the present disclosure to an administration subject. When bone formation is carried out *in vivo* by the bone-forming method of the present disclosure, the administration position of the bone-forming composition of the present disclosure can be a desired position where bone formation is to be carried out. In the bone-forming method of the present disclosure, for the administration subject and the administration conditions, the descriptions of the administration subject and the administration conditions of the bone-forming composition can be applied.

In the bone-forming method of the present disclosure, the bone-forming kit of the present disclosure may be used as the bone-forming composition of the present disclosure.

### <Use>

The present disclosure includes a use of a bone-forming composition comprising enlarged megakaryocytes and/or an extracellular secretion product derived from enlarged megakaryocytes, for use in bone formation. The present disclosure includes a use of a bone-forming composition comprising enlarged megakaryocytes and/or an extracellular secretion product derived from enlarged megakaryocytes, for producing a bone-forming composition. For the present disclosure, for example, the descriptions of the bone-forming composition, the bone-forming kit, and the bone-forming method of the present disclosure can be applied.

### EXAMPLES

Hereinafter, the present disclosure will be described in detail using Examples, but the present disclosure is not limited to the embodiments described in the Examples.

### [Example 1]

A bone-forming composition of the present disclosure was produced, and it was confirmed that the bone-forming composition of the present disclosure exhibits a bone-forming effect.

### (1) Production of Immortalized Megakaryocytes

Immortalized megakaryocytes were produced by the following procedure. As the pre-multinucleated megakaryocytes, immortalized megakaryocytes were used. The immortalized megakaryocytes were produced by the following procedure.

### (1-1) Preparation of Hematopoietic Progenitor Cells from iPS Cells

Human iPS cells were subjected to differentiation culture into blood cells according to the method described in Reference 6 below. Specifically, human iPS cell colonies maintained on laminin 511-E8 (Nippi) were cultured overnight in the presence of 50 ng/ml activin A (WAKO) and 3 µM CHIR99021 (WAKO), and then co-cultured with C3H10T1/2 feeder cells for 14 days in the presence of 20 ng/mL VEGF (R&D SYSTEMS) to produce hematopoietic progenitor cells (HPCs). As for the culture conditions, the maintenance of the iPS cells and the first 7 days of the HPC induction were carried out at 37°C, 5% O₂, and 5% CO₂, and thereafter carried out at 37°C, 20% O₂, and 5% CO₂.

### Reference 6: Takayama N. et al., "Transient activation of c-MYC expression is critical for efficient platelet generation from human induced pluripotent stem cells", J. Exp. Med., 2010, vo.13, pages 2817-2830

### (1-2) Gene Transduction System

As the gene transduction system, a lentiviral vector system was used. As the lentiviral vector, a tetracycline-controlled Tet-on (registered trademark) gene expression induction system vector was used. The vectors were vectors into which *c-MYC*, *BMI1*, or *BCL-XL* was introduced under a TRE promoter, and the respective vectors were constructed as EN-TRE-c-Myc-Ubc-rtTA-KR, EN-TRE-BMI1-Ubc-rtTA-KR, and EN-TRE-BCL-XL-Ubc-rtTA-KR. Next, viruses containing the c-MYC, BMI1, and BCL-XL vectors were prepared by transducing the lentiviral vectors into 293T cells (the aforementioned Reference 2 and Reference 7 below). Then, by infecting the target cells with the obtained viruses, the c-MYC, BMI1, and BCL-XL genes can be introduced into the genomic sequence of the target cells. In addition, when the c-MYC, BMI1, and BCL-XL genes are stably introduced into the genomic sequence in the obtained cells, the cells can forcibly express the c-MYC, BMI1, and BCL-XL genes by adding doxycycline (clontech #631311) to the medium.

### Reference 7: Yamaguchi et al., "Development of an All-in-One Inducible Lentiviral Vector for Gene Specific Analysis of Reprogramming.", PLoS ONE, 2012, vol.7 (7) e41007

### (1-3)Induction of Megakaryocyte Cell Line by Introduction of Three Genes into Hematopoietic Progenitor Cells

The HPCs produced in the above Example 1 (1-1) were infected with the three kinds of lentiviruses produced in the above Example 1 (1-2) to induce megakaryocyte progenitor cells. Approximately 25 days after the infection, strains having sufficient proliferative capacity were immunostained using an anti-human CD41a-APC antibody (BioLegend), an anti-human CD42b-PE antibody (eBioscience), and an anti-human CD235ab-Pacific Blue (Anti-CD235ab-PB; BioLegend) antibody, and then analyzed using a FACS Verse (trademark). Strains in which both the CD41a-positive fraction and the CD42b-positive fraction were 50% or more were designated as immortalized megakaryocyte cell lines (MKCLs, corresponding to pre-multinucleated megakaryocytes) and were used in the subsequent studies.

### (1-4) Proliferation Culture of Megakaryocyte Cell Line

The proliferation culture of the obtained MKCLs was carried out in a 10 cm dish (10 ml/dish) or a 125 ml shaking flask. As the medium, IMDM (Sigma Aldrich #I3390) was used as a basal medium, and the following components were added (concentrations are final concentrations; proliferation medium). The culture conditions were 37°C and 5% CO₂, and the shaking speed during shaking was set to 100 rpm using a shaker with a rotational diameter of 19 mm.
FBS (Hyclone #SH30071.03, fetal bovine serum) 15%
Glutamax (GIBCO #3505-061) 2 mmol/l
ITS-G (Gibco #41400-045) 100-fold dilution
MTG (monothioglycerol, FUJIFILM Wako Pure Chemical #195-157) 450 µmol/l
Ascorbic acid (NIPRO #49871900040329) 50 µg/ml
SCF (Wako Pure Chemical #193-15513) 50 ng/ml
TPO-like agent 200 ng/mL
Doxycycline (DOX) (clontech #631311) 1 µg/ml

### (2) Induction of Multinucleation of Immortalized Megakaryocytes

Next, multinucleation of the immortalized megakaryocytes was induced by culturing in a medium to which, in addition to DOX, an AhR inhibitor, a ROCK inhibitor, a myosin 2 inhibitor, and harmine were added. Specifically, the immortalized megakaryocyte cell line obtained in the above Example 1 (1) was suspended in the following multinucleation medium so as to reach 3.0×10⁵ cells/ml. Then, the suspension was seeded in a 125 ml Erlenmeyer flask at 25 ml/flask and cultured with shaking at 100 rpm for 3 days. The culture conditions were 37°C and 5% CO₂.

### (Multinucleation Medium)

Human AB serum 10% (AccessBiologicals)
Glutamax (GIBCO #3505-061) 2 mmol/L
ITS-G (Gibco #41400-045) 100-fold dilution
MTG (monothioglycerol, FUJIFILM Wako Pure Chemical #195-157) 450 µmol/l
Ascorbic acid (NIPRO #49871900040329) 50 µg/ml
SCF (Wako Pure Chemical #193-15513) 50 ng/ml
TPO-like agent 200 ng/ml
Doxycycline (clontech #631311) 1 µg/ml
Aryl hydrocarbon receptor (AhR) inhibitor GNF351 (Calbiochem #182707) 500 nmol/l
ROCK inhibitor Y-39983 (Medchemexpress #MCH-HY-13300-10) 500 nmol/l
Harmine (Sigma Aldrich #286044-1G) 5 µmol/l
Myosin 2 inhibitor (-)-Blebbistatin (Medchem Express #HY-13441) 10 µmol/l

### (3) Induction of Enlarged Megakaryocytes

Next, by further culturing the multinucleated megakaryocytes, enlarged megakaryocytes were induced from the multinucleated megakaryocytes. Specifically, the multinucleated megakaryocytes obtained in the above Example 1 (2) were suspended in the following enlargement medium so as to reach 1.0×10⁵ cells/ml. Then, the suspension was seeded in a 125 ml Erlenmeyer flask at 25 ml/flask and cultured with shaking under conditions of 100 rpm for 2 days or 3 days. The culture conditions were 37°C and 5% CO₂. Next, the cultured medium containing the enlarged megakaryocytes was centrifuged under conditions of 300×g for 5 minutes, and the precipitate was suspended in 30 ml of a washing/preservation solution (2.5% human serum albumin, 20% ACD-A solution, BICANATE Injection (pH 7.3)). After the suspension, X-ray irradiation was performed at 25 Gy to inhibit the proliferation of the enlarged megakaryocytes. After the physical stimulation, the enlarged megakaryocytes were stored at 4°C.

### (Enlargement Medium)

Human AB serum 10% (AccessBiologicals)
Glutamax (GIBCO #3505-061) 2 mmol/L
ITS-G (Gibco #41400-045) 100-fold dilution
MTG (monothioglycerol, FUJIFILM Wako Pure Chemical #195-157) 450 µmol/l
Ascorbic acid (NIPRO #49871900040329) 50 µg/ml
SCF (Wako Pure Chemical #193-15513) 50 ng/ml
TPO-like agent 200 ng/ml
Aryl hydrocarbon receptor (AhR) inhibitor GNF351 (Calbiochem #182707) 500 nmol/l
ROCK inhibitor Y-39983 (Medchemexpress #MCH-HY-13300-10) 500 nmol/l
Enoxaparin (Sanofi) 1 U/mL

### (4) Study of Bone Formation and Bone Augmentation at Calvarial Bone Defect Site in vivo

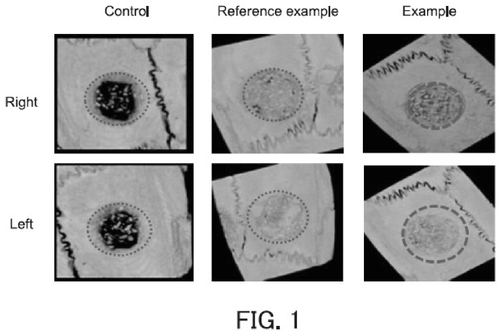

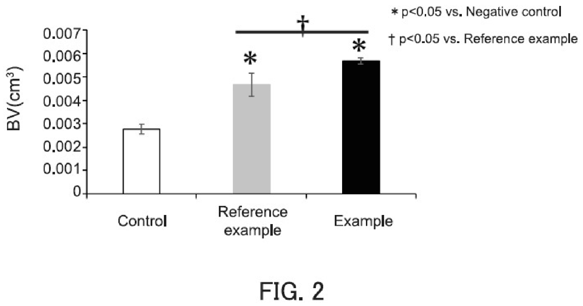

It was studied whether the enlarged megakaryocytes of the present disclosure contribute to bone formation and bone augmentation at a calvarial bone defect site *in vivo*. Specifically, first, sample preparation was performed using the enlarged megakaryocytes (cultured for 2 days) obtained in the above Example 1 (3). A cell suspension containing the enlarged megakaryocytes was centrifuged under conditions of 300×g for 5 minutes. After the centrifugation, the enlarged megakaryocytes were suspended in physiological saline to prepare a preparation solution. After the preparation, a preparation solution equivalent to 1.0×10⁶ cells was added to an artificial bone material (octacalcium phosphate (OCP)/Collagen (Col), Bonarc^{®}). Next, a defect site having a diameter of 4 mm was created in the calvaria of nude rats (11 weeks old) under general anesthesia. The artificial bone material was transplanted onto the defect site and onto the calvaria other than the defect site (Example). For the negative control group, transplantation was performed by the same method except that the artificial bone material impregnated with PBS was used. In addition, for the reference example, transplantation was performed by the same method except that the artificial bone material impregnated with a composition (25 mg/ml iMDF) prepared from a platelet-depleted megakaryocyte culture was used. The method for producing the platelet-depleted megakaryocyte culture was prepared with reference to the method described in WO 2022/092169, using the same number of megakaryocytes. Four weeks after the transplantation, the artificial bone material including the surrounding calvaria was excised, and the obtained excised portion was subjected to micro-computed tomography (micro-CT) imaging. In addition, the bone volume of the bone newly formed from the artificial bone material (newly formed bone) was evaluated. In the evaluation, the bone volume (BV (cm³)) was measured using 3D bone analysis software (TRI/3D-BON; Ratoc System Engineering). These results are shown in FIG. 1 and FIG. 2.

FIG. 1 is a photograph showing bone formation 4 weeks after the transplantation. In FIG. 1, from left to right, are photographs showing the results of the negative control group, the reference example, and the Example. In FIG. 1, the upper row is a photograph showing the results of the right calvaria of the defect site, and the lower row is a photograph showing the left calvaria of the defect site. As illustrated in FIG. 1, compared with the negative control group, in the reference example and the Example, bone was newly formed at the defect site of the calvaria.

FIG. 2 is a graph showing bone volume 4 weeks after the transplantation. In FIG. 2, the vertical axis represents the bone volume (BV (cm³)), and the horizontal axis represents the type of experimental group. As illustrated in FIG. 2, compared with the negative control group, the bone volume increased in the reference example and the Example. In addition, compared with the reference example, the bone volume increased in the Example. From these results, it was found that the composition containing the enlarged megakaryocytes of the present disclosure can induce and promote bone formation.

### (5) Preparation of Secretome Derived from Enlarged Megakaryocytes

Using enlarged megakaryocytes, a secretome derived from enlarged megakaryocytes was prepared. To 300 ml of a cultured medium containing from 1 to 2×10⁵ cells/ml of the enlarged megakaryocytes (cultured for 2 days or 3 days) obtained in the above Example 1 (3), citric acid (ACD-A) was added, and centrifugation was performed under conditions of 2000×g for 20 minutes. After the centrifugation, the enlarged megakaryocytes were collected and suspended in citric acid/Ringer's solution. After the suspension, centrifugation was performed under conditions of 2000×g for 20 minutes. After the centrifugation, the enlarged megakaryocytes were collected, suspended in 15 to 20 ml of DMEM, and adjusted to from 1 to 2×10⁶ cells/ml. Thereafter, the enlarged megakaryocytes were subjected to physical stimulation using 60 Gy of X-ray irradiation and glass beads (Cellaid, JMS Co., Ltd.). After the physical stimulation, centrifugation was performed under conditions of 2000×g for 20 minutes. After the centrifugation, filtration was performed. For the filtration, a 0.8 µm filter and a 0.22 µm filter were used. After the filtration, a secretome preparation solution derived from enlarged megakaryocytes was obtained by collecting the cultured medium concentrated by ultrafiltration at 100k. Hereinafter, the secretome derived from enlarged megakaryocytes cultured for 2 days is also referred to as Day2 secretome, and the secretome derived from enlarged megakaryocytes cultured for 3 days is also referred to as Day3 secretome.

### (6) Study of Bone Formation and Bone Augmentation at Calvarial Bone Defect Site by Secretome Derived from Enlarged Megakaryocytes in vivo

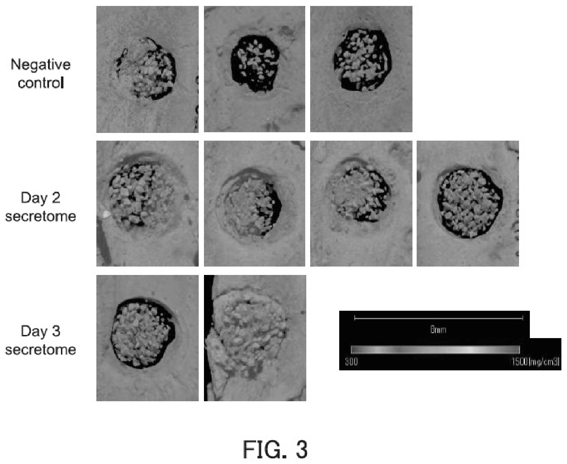

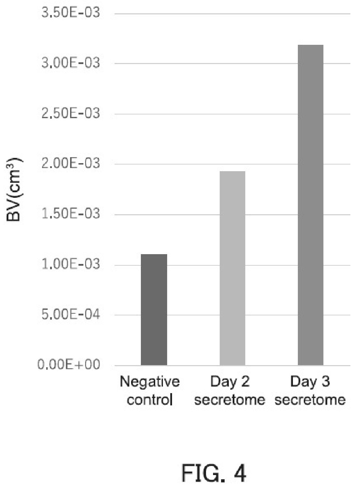

It was studied whether the secretome derived from enlarged megakaryocytes of the present disclosure contributes to bone formation and bone augmentation at a calvarial bone defect site *in vivo*. Specifically, an artificial bone material was transplanted into wild-type rats by the same method as in the above Example 1 (4), except that, instead of the enlarged megakaryocytes, 20 µl of the Day2 secretome or Day3 secretome (each equivalent to from 1.6×10⁵ to 1.8×10⁵ cells) obtained in the above Example 1 (5) was used. In addition, for the negative control group, transplantation was performed by the same method except that the artificial bone material impregnated with PBS was used. Four weeks after the transplantation, the artificial bone material including the surrounding calvaria was excised, and the obtained excised portion was subjected to micro-CT imaging. In addition, the bone volume of the bone newly formed from the artificial bone material (newly formed bone) was evaluated. In the evaluation, the bone volume (BV (cm³)) was measured using 3D bone analysis software (TRI/3D-BON; Ratoc System Engineering). These results are shown in FIG. 3 and FIG. 4.

FIG. 3 is a photograph showing bone formation 4 weeks after the transplantation. In FIG. 3, the upper row shows the negative control group, the middle row shows the group using the Day2 secretome, and the lower row is a photograph showing the results of the group using the Day3 secretome. As illustrated in FIG. 3, compared with the negative control group, in the group using the Day2 secretome and the group using the Day3 secretome, bone was newly formed at the defect site of the calvaria.

FIG. 4 is a graph showing bone volume 4 weeks after the transplantation. In FIG. 4, the vertical axis represents the bone volume (BV (cm³)), and the horizontal axis represents the type of experimental group. As illustrated in FIG. 4, compared with the negative control group, the bone volume increased in the group using the Day2 secretome and the group using the Day3 secretome. From these results, it was found that the composition containing the secretome derived from enlarged megakaryocytes of the present disclosure can induce and promote bone formation.

### (7) Action of Secretome Derived from Enlarged Megakaryocytes on Bone Marrow-Derived Mesenchymal Stem Cells

The action of the secretome derived from enlarged megakaryocytes of the present disclosure on bone marrow-derived mesenchymal stem cells was studied. Specifically, 4.0×10⁴ cells of bone marrow-derived mesenchymal stem cells (MSCs) were seeded in a 24-well plate and cultured until 80% confluence. After the culture, the Day3 secretome obtained in the above Example 1 (5) or an osteoblast differentiation induction medium (DGA) was added. The DGA was prepared by adding 10% FBS, 50 mg/ml ascorbic acid, 10 mmol/l beta-glycerophosphate, and 100 nmol/l dexamethasone to DMEM (low glucose).

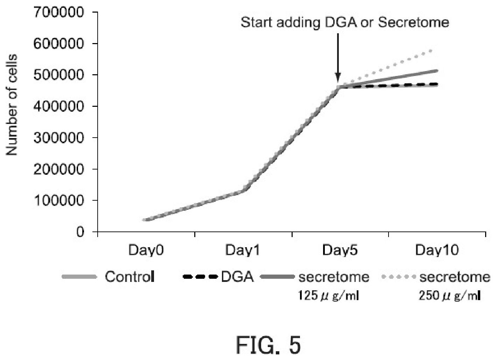

Using the culture start date as a reference, the cell number was measured on day 0, 1, 5, or 10. The cell number was calculated using a cell counting kit (WST-8, Cell Counting Kit-8, Dojindo, Cat No. 347-07621) according to the attached protocol. These results are shown in FIG. 5.

FIG. 5 is a graph showing the cell number. In FIG. 5, the horizontal axis represents the number of days after the start of the culture of the bone marrow-derived mesenchymal stem cells, and the vertical axis represents the cell number. As illustrated in FIG. 5, whereas the increase in the cell number of the bone marrow-derived mesenchymal stem cells was inhibited and remained flat when the DGA was added, the cell number of the bone marrow-derived mesenchymal stem cells increased when the Day3 secretome was added. In addition, it was found that the cell number of the bone marrow-derived mesenchymal stem cells increased in a manner dependent on the concentration of the Day3 secretome. From these results, it was found that the secretome derived from enlarged megakaryocytes of the present disclosure promotes the proliferation of bone marrow-derived mesenchymal stem cells.

Although the present disclosure has been described above with reference to the embodiments and Examples, the present disclosure is not limited to the above embodiments and Examples. Various changes that can be understood by those skilled in the art within the scope of the present disclosure can be made to the configuration and details of the present disclosure.

This application claims priority based on Japanese Patent Application No. 2024-019962 filed on February 14, 2024, the entire disclosure of which is incorporated herein by reference.

### <Supplementary Notes>

Some or all of the above embodiments and Examples may be described as in the following supplementary notes, but are not limited to the following.

### <Bone-Forming Composition>

### (Supplementary Note 1)

A bone-forming composition comprising enlarged megakaryocytes and/or extracellular secretion products derived from enlarged megakaryocytes.

### (Supplementary Note 2)

The bone-forming composition according to Supplementary Note 1, wherein
the enlarged megakaryocytes comprise alpha-granules.

### (Supplementary Note 3)

The bone-forming composition according to Supplementary Note 1 or 2, wherein the enlarged megakaryocytes have a corresponding expression level of Ki-67 and/or MYC that is equal to or less than 50% relative to the expression level of Ki-67 and/or MYC in multinucleated megakaryocytes.

### (Supplementary Note 4)

The bone-forming composition according to any one of Supplementary Notes 1 to 3, wherein:
the enlarged megakaryocytes are a cell population comprising the enlarged megakaryocytes;
the enlarged megakaryocytes comprise enlarged megakaryocytes having a ploidy of 16N or more, and;
in the cell population comprising the enlarged megakaryocytes, aproportion (cell number) of the enlarged megakaryocytes of 16N or more is 20% or more.

### (Supplementary Note 5)

The bone-forming composition according to Supplementary Note 4, wherein
in the cell population, the proportion (cell number) of the enlarged megakaryocytes of 16N or more is 20% to 90%.

### (Supplementary Note 6)

The bone-forming composition according to Supplementary Note 5, wherein,
in the cell population, the proportion (cell number) of the enlarged megakaryocytes of 16N or more is 20% to 70%.

### (Supplementary Note 7)

The bone-forming composition according to any one of Supplementary Notes 1 to 6, wherein:
the enlarged megakaryocytes are a cell population comprising the enlarged megakaryocytes,
the enlarged megakaryocytes comprise enlarged megakaryocytes having a ploidy of 32N or more; and,
in the cell population, the proportion (cell number) of the enlarged megakaryocytes of 32N or more is 5% to 50%.

### (Supplementary Note 8)

The bone-forming composition according to Supplementary Note 7, wherein:
the enlarged megakaryocyte comprises an enlarged megakaryocyte of 32N or more: and,
in the cell population, the proportion (cell number) of the enlarged megakaryocytes having a ploidy of 32N or more is 5% to 40%.

### (Supplementary Note 9)

The bone-forming composition according to any one of Supplementary Notes 1 to 8, wherein
the enlarged megakaryocytes are produced by a production method comprising:
a multinucleation step of multinucleating pre-multinucleated megakaryocytes or progenitor cells thereof to induce multinucleated megakaryocytes; and
an enlargement step of maturing the multinucleated megakaryocytes to induce enlarged megakaryocytes, wherein,
in the multinucleation step, the pre-multinucleated megakaryocytes are multinucleated to induce multinucleated megakaryocytes with inhibiting at least one activity selected from the group consisting of aryl hydrocarbon receptor (AhR), Rho-associated coiled-coil forming kinase (ROCK), dual-specificity tyrosine phosphorylation-regulated kinase (DYRK), and myosin 2, and in the presence of harmine.

### (Supplementary Note 10)

The bone-forming composition according to Supplementary Note 9, wherein
in the multinucleation step, the pre-multinucleated megakaryocytes or the progenitor cells thereof are cultured and multinucleated to induce a multinucleated megakaryocyte in the presence of an AhR inhibitor, a myosin 2 inhibitor, and harmine.

### (Supplementary Note 11)

The bone-forming composition according to Supplementary Note 9 or 10, wherein
the production method comprises a proliferation step of proliferating the pre-multinucleated megakaryocytes, and
in the proliferation step, the pre-multinucleated megakaryocytes are proliferated without inhibiting the activities of AhR, ROCK, DYRK, and myosin 2, and in the absence of harmine.

### (Supplementary Note 12)

The bone-forming composition according to Supplementary Note 11, wherein
the multinucleation step is carried out after the proliferation step.

### (Supplementary Note 13)

The bone-forming composition according to any one of Supplementary Notes 1 to 12, comprising a cultured medium of the enlarged megakaryocytes, wherein
the cultured medium comprises the enlarged megakaryocytes and/or the extracellular secretion products derived from enlarged megakaryocytes.

### (Supplementary Note 14)

The bone-forming composition according to Supplementary Note 13, comprising a concentrate of the cultured medium.

### (Supplementary Note 15)

The bone-forming composition according to any one of Supplementary Notes 1 to 14, comprising isolated extracellular vesicles.

### (Supplementary Note 16)

The bone-forming composition according to any one of Supplementary Notes 1 to 15, wherein
the enlarged megakaryocytes are enlarged megakaryocytes induced *in vitro*.

### (Supplementary Note 17)

The bone-forming composition according to any one of Supplementary Notes 1 to 16, wherein
the enlarged megakaryocytes are derived from immortalized megakaryocytes.

### (Supplementary Note 18)

The bone-forming composition according to any one of Supplementary Notes 1 to 17, wherein
the enlarged megakaryocytes comprise an exogenous oncogene, polycomb gene, and/or anti-apoptotic gene.

### (Supplementary Note 19)

The bone-forming composition according to any one of Supplementary Notes 1 to 18, wherein
the composition has an activity of promoting proliferation of a cell.

### (Supplementary Note 20)

The bone-forming composition according to Supplementary Note 19, wherein
the cell is a mesenchymal stem cell.

### (Supplementary Note 21)

The bone-forming composition according to Supplementary Note 20, wherein
the mesenchymal stem cell is a progenitor cell of an osteoblast.

### <Bone-Forming Kit>

### (Supplementary Note 22)

A bone-forming kit comprising a bone-forming composition and a scaffold material for bone formation, wherein
the bone-forming composition is the bone-forming composition according to any one of Supplementary Notes 1 to 21.

### (Supplementary Note 23)

The bone-forming kit according to Supplementary Note 22, wherein
the scaffold material exhibits biocompatibility and/or biodegradability.

### (Supplementary Note 24)

The kit according to Supplementary Note 22 or 23, wherein
the scaffold material is composed of at least one selected from the group consisting of collagen, gelatin, albumin, keratin, dextran, carboxymethyl cellulose, xanthan gum, chitosan, chondroitin sulfate, heparin, hyaluronic acid, polyglycolic acid, polylactic acid, a copolymer of polyglycolic acid and polylactic acid, polyhydroxybutyrate, polydioxanone, polycaprolactone, polybutylene succinate, calcium phosphate, hydroxyapatite, polyether ketone, and polyether ether ketone.

### (Supplementary Note 25)

The kit according to any one of Supplementary Notes 22 to 24, wherein
the scaffold material has a porous structure.

### (Supplementary Note 26)

The kit according to any one of Supplementary Notes 22 to 25, comprising a physiologically active substance.

### (Supplementary Note 27)

The kit according to any one of Supplementary Notes 22 to 26, comprising at least one selected from the group consisting of vascular endothelial growth factor (VEGF), platelet-derived growth factor (PDGF), epidermal growth factor (EGF), fibroblast growth factor (FGF), hepatocyte growth factor (HGF), insulin-like growth factor (IGF), brain-derived neurotrophic factor (BDNF), growth differentiation factor-5 (GDF5), erythropoietin (EPO), transforming growth factor (TGF), and bone morphogenetic protein.

### <Bone-Forming Method>

### (Supplementary Note 28)

A bone-forming method using the bone-forming composition according to any one of Supplementary Notes 1 to 21.

### (Supplementary Note 29)

The bone-forming method according to Supplementary Note 28, comprising an administration step of administering the bone-forming composition to a subject.

### (Supplementary Note 30)

The bone-forming method according to Supplementary Note 28 or 29, wherein
the bone-forming composition is used *in vitro* or *in vivo*.

### <Use>

### (Supplementary Note 31)

Use of the bone-forming composition according to any one of Supplementary Notes 1 to 21 for bone formation.

### INDUSTRIAL APPLICABILITY

As described above, according to the present disclosure, a bone-forming composition comprising secretomes and/or extracellular vesicles derived from enlarged megakaryocytes can be provided. Therefore, the present disclosure is extremely useful, for example, in the field of regenerative medicine, the field of cosmetics, and the like.

## Claims

1. A bone-forming composition comprising enlarged megakaryocytes and/or extracellular secretion products derived from enlarged megakaryocytes.

2. The bone-forming composition according to claim 1,
wherein the enlarged megakaryocytes comprise alpha-granules.

3. The bone-forming composition according to claim 1 or 2,
wherein the enlarged megakaryocytes have a corresponding expression level of Ki-67 and/or MYC that is equal to or less than 50% relative to an expression level of Ki-67 and/or MYC in multinucleated megakaryocytes.

4. The bone-forming composition according to any one of claims 1 to 3,
wherein the enlarged megakaryocytes are a cell population comprising the enlarged megakaryocytes:
the enlarged megakaryocytes comprise enlarged megakaryocytes having a ploidy of 16N or more: and,
in the cell population comprising the enlarged megakaryocytes, a proportion (cell number) of the enlarged megakaryocytes of 16N or more is 20% or more.

5. The bone-forming composition according to any one of claims 1 to 4,
wherein the enlarged megakaryocytes are a cell population comprising the enlarged megakaryocytes:
the enlarged megakaryocytes comprise enlarged megakaryocytes having a ploidy of 32N or more: and,
in the cell population, a proportion (cell number) of the enlarged megakaryocytes of 32N or more is 5% to 50%.

6. The bone-forming composition according to any one of claims 1 to 5,
wherein the enlarged megakaryocytes are produced by a production method comprising a multinucleation step and an enlargement step,
in the multinucleation step, pre-multinucleated megakaryocytes or progenitor cells thereof are multinucleated to induce multinucleated megakaryocytes, and
in the enlargement step, the multinucleated megakaryocytes are matured to induce the enlarged megakaryocytes, and
in the multinucleation step, the pre-multinucleated megakaryocytes are multinucleated to induce multinucleated megakaryocytes with inhibiting at least one activity selected from the group consisting of aryl hydrocarbon receptor (AhR), Rho-associated coiled-coil forming kinase (ROCK), dual-specificity tyrosine phosphorylation-regulated kinase (DYRK), and myosin 2, and in the presence of harmine.

7. The bone-forming composition according to claim 6,
wherein in the multinucleation step, the pre-multinucleated megakaryocytes or the progenitor cells thereof are cultured and multinucleated to induce a multinucleated megakaryocytes in the presence of an AhR inhibitor, a myosin 2 inhibitor, and harmine.

8. The bone-forming composition according to claim 6 or 7,
wherein the production method comprises a proliferation step, and
in the proliferation step, the pre-multinucleated megakaryocytes are proliferated without inhibiting the activities of AhR, ROCK, DYRK, and myosin 2 and in the absence of harmine.

9. The bone-forming composition according to claim 8,
wherein the multinucleation step is performed after the proliferation step.

10. The bone-forming composition according to any one of claims 1 to 9, comprising a cultured medium of the enlarged megakaryocytes,
wherein the cultured medium comprises the enlarged megakaryocytes and/or the extracellular secretion products derived from enlarged megakaryocytes.

11. The bone-forming composition according to claim 10, comprising a concentrate of the cultured medium.

12. The bone-forming composition according to any one of claims 1 to 11, comprising isolated extracellular vesicles.

13. The bone-forming composition according to any one of claims 1 to 12,
wherein the enlarged megakaryocytes are enlarged megakaryocytes induced *in vitro*.

14. The bone-forming composition according to any one of claims 1 to 13,
wherein the enlarged megakaryocytes are derived from immortalized megakaryocytes.

15. The bone-forming composition according to any one of claims 1 to 14,
wherein the enlarged megakaryocytes comprise an exogenous oncogene, polycomb gene, and/or anti-apoptotic gene.

16. The bone-forming composition according to any one of claims 1 to 15,
wherein the composition has an activity of promoting proliferation of a cell.

17. The bone-forming composition according to claim 16,
wherein the cell is a mesenchymal stem cell (MSC).

18. A bone-forming kit comprising:
a bone-forming composition; and
a scaffold material for bone formation,
wherein the bone-forming composition is the bone-forming composition according to any one of claims 1 to 17.

19. The bone-forming kit according to claim 18,
wherein the scaffold material exhibits biocompatibility and/or biodegradability.

20. The kit according to claim 18 or 19,
wherein the scaffold material is composed of at least one selected from the group consisting of collagen, gelatin, albumin, keratin, dextran, carboxymethyl cellulose, xanthan gum, chitosan, chondroitin sulfate, heparin, hyaluronic acid, polyglycolic acid, polylactic acid, a copolymer of polyglycolic acid and polylactic acid, polyhydroxybutyrate, polydioxanone, polycaprolactone, polybutylene succinate, calcium phosphate, hydroxyapatite, polyether ketone, and polyether ether ketone.
